(19) Europäisches Patentamt
European Patent Office
Office européen des brevets

(11) Veröffentlichungsnummer: **0 147 768 B1**

(12) # EUROPÄISCHE PATENTSCHRIFT

(45) Veröffentlichungstag der Patentschrift: **08.02.89**

(21) Anmeldenummer: **84115698.7**

(22) Anmeldetag: **18.12.84**

(51) Int. Cl.⁴: **C 07 D 213/04**, C 07 D 277/22, C 07 D 233/64, C 07 C 125/065, C 07 C 125/073, C 07 C 69/18, C 07 C 69/96, C 07 C 69/003, C 07 C 103/167, C 07 C 69/63, C 07 C 103/38

(54) **Glycerinderivate.**

(30) Priorität: **30.12.83 CH 6985/83**
**20.09.84 CH 4498/84**

(43) Veröffentlichungstag der Anmeldung: **10.07.85 Patentblatt 85/28**

(45) Bekanntmachung des Hinweises auf die Patenterteilung: **08.02.89 Patentblatt 89/6**

(84) Benannte Vertragsstaaten: **AT BE CH DE FR GB IT LI LU NL SE**

(56) Entgegenhaltungen:
**EP-A- 0 146 258**
**DD-A- 121 507**
**DE-A- 1 954 718**
**DE-A- 2 422 170**
**DE-A- 2 820 892**
**DE-B- 1 199 254**
**DE-C- 932 611**
**US-A- 3 671 557**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Barner, Richard, Dr., Traubenweg 16, CH-4108 Witterswil (CH)**
Erfinder: **Hadvary, Paul, Dr., Neumattenweg 8, CH-4105 Biel-Benken (CH)**
Erfinder: **Burri, Kaspar, Dr., Im Lichs 3, CH-4310 Rheinfelden (CH)**
Erfinder: **Hirth, Georges, 7 rue de l'ancre, F-68330 Huningue (FR)**
Erfinder: **Cassal, Jean-Marie, Dr., 4 rue du Markstein, F-68100 Mulhouse (FR)**
Erfinder: **Müller, Klaus, Dr., Grellingerstrasse 5, CH-4142 Münchenstein (CH)**

(74) Vertreter: **Lederer, Franz, Dr. et al, Van der Werth, Lederer & Riederer Patentanwälte Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Glycerinderivate, Verfahren zu ihrer Herstellung, neue Zwischenprodukte dazu und Arzneimittel auf der Basis jener Glycerinderivate.

Die erfindungsgemässen Glycerinderivate sind Verbindungen der Formel

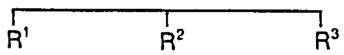

I

worin einer der Reste $R^1$, $R^2$ und $R^3$ eine Gruppe U der Formel $OY^1$ oder $X^1$–C(O)–$(A^1)_n$–$Z^1$,

ein weiterer eine Gruppe V der Formel $OY^2$ oder $X^2$–C(O)–$(A^2)_p$–$Z^2$,

und der verbleibende eine Gruppe W der Formel $X^3$T–($C_{2-6}$-Alkylen)–N(Het)$Q^-$ ist,

wobei eines von $X^1$, $X^2$ und $X^3$ Sauerstoff oder NH ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

$A^1$ und $A^2$ Sauerstoff oder NH,

n und p die Zahl 1 oder 0,

$Z^1$ $C_{9-25}$-Alkyl oder $C_{9-25}$-Alkenyl,

$Z^2$ $C_{1-5}$-Alkyl, $C_{2-5}$-Alkenyl, Phenyl oder, falls $(A^2)_p$ nicht Sauerstoff ist, auch Wasserstoff bedeutet,

T Carbonyl, C(O)O oder C(O)NH oder, falls $X^3$ Sauerstoff ist, auch Methylen bedeutet, wobei, falls $R^1$ eine Gruppe $OY^1$ oder $OY^2$ und gleichzeitig $R^2$ eine Gruppe $OY^1$ oder V und $X^3$ Sauerstoff ist, T eine Gruppe C(O)O oder C(O)NH sein soll,

$-\overset{+}{N}$(Het) ein gegebenenfalls durch Hydroxy, $C_{1-4}$-Alkyl, $C_{1-4}$-Alkoxy, 2-(Hydroxy oder Amino)-äthyl, Carbamoyl oder Ureido mono-substituierter Oxazolium-, Isoxazolium-, Pyridazinium, Chinolinium-, Isochinolinium-, N-Methylimidazolium, Pyridinium- oder Thiazoliumrest, und $Q^-$ das Anion einer starken organischen oder anorganischen Säure ist,

und deren Hydrate.

Die hier verwendeten Ausdrücke «Alkyl» und «Alkenyl» beziehen sich auf geradkettige oder verzweigte, gesättigte bzw. monoungesättigte Reste, wie Methyl, Äthyl, Propyl, Isopropyl, 2-Propenyl, Butyl, Isobutyl, Hexadecyl, Heptadecyl, Octadecyl und Octadecenyl, insbesondere Methyl und Octadecyl. Beispiele von $C_{3-6}$-Cycloalkylresten $Y^2$ sind Cyclopropyl und Cyclohexyl, von $C_{5-6}$-Cycloalkenylresten $Y^2$ 2-Cyclopentenyl und insbesondere 2-Cyclohexenyl. $C_{2-6}$-Alkylengruppen können geradkettig oder verzweigt sein. Beispiele davon sind n-Butylen, 2-Methylpropylen und insbesondere Äthylen und Propylen. Beispiele von heterocyclischen Resten –N(Het) sind Oxazolium, Isoxazolium, Pyridazinium, Chinolinium, Isochinolinium und N-Methylimidazolium und insbesondere Pyridinium und Thiazolium.

Beispiele für Anionen starker organischer oder anorganischer Säuren sind $C_{1-4}$-Alkylsulfonyloxy, Phenylsulfonyloxy, Tosyloxy, Campher-10-sulfonyloxy bzw. $Cl^-$, $Br^-$, $J^-$, $ClO_4^{--}$, $SO_4^{--}$, $PO_4^{---}$ und $NO_3^-$.

Die Verbindungen der Formel I können hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Verbindungen der Formel I enthalten zumindest ein asymmetrisches C-Atom und können somit als optisch aktive Enantiomere, als Diastereomere oder als Gemische davon, z. B. als Racemate, vorliegen.

Bevorzugte Verbindungen der Formel I sind diejenigen, worin $R^3$ eine Gruppe W ist.

Bevorzugte Verbindungen der Formel I sind ferner diejenigen, worin $R^1$ Octadecylcarbamoyloxy, $R^2$ Methoxyformamido, Methoxy oder insbesondere Methoxycarbonyloxy und/oder worin $R^3$ 4-(1-Pyridiniumchlorid)-n-butyryloxy, 4-(1-Pyridiniumjodid)-n-butyryloxy, 4-(3-Thiazoliumchlorid)-n-butyryloxy oder insbesondere 4-(3-Thiazoliumjodid)-n-butyryloxy ist.

Beispiele solcher Verbindungen sind insbesondere

das 3-[3-[[(R)-2-(Methoxycarbonyloxy)-3-[(octadecylcarbamoyl)oxy]propoxy]carbonyl]propyl]thiazoliumjodid, sowie

das 3-[3-[[(S)-2-[(Methoxycarbonyl)oxy]-3-[(octadecylcarbamoyl)oxy]propoxy]carbonyl]propyl]thiazoliumjodid,

das 1-[3-[[(RS)-2-[(Methoxycarbonyl)oxy]-3-[(octadecylcarbamoyl)oxy]propoxy]carbonyl]-propyl]pyridiniumchlorid,

das 1-[3-[[(RS)-2-(1-Methoxyformamido-3-(octadecylcarbamoyloxy)propoxy]carbonyl]propyl]thiazoliumchlorid,

das 1-[3-[[(S)-2-[(Methoxycarbonyl)oxy]-3-[(octadecylcarbamoyl)oxy]propoxy]carbonyl]propyl]pyridiniumjodid,

das 1-[3-[[(R)-2-[(Methoxycarbonyl)oxy]-3-[(octadecylcarbamoyl)oxy]propoxy]carbonyl]propyl]pyridiniumchlorid und

das 3-[3-[[(RS)-2-Methoxy-3-[(octadecylcarbamoyl)oxy]propoxy]carbonyl]propyl]thiazoliumchlorid.

Die Verbindungen der Formel I und ihre Hydrate können dadurch hergestellt werden, dass man

a) eine Verbindung der Formel

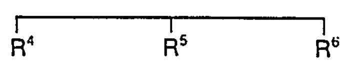

II

worin die Reste $R^4$, $R^5$ und $R^6$ die gleiche Bedeutung wie die Reste $R^1$, $R^2$ bzw. $R^3$ haben, wobei jedoch an Stelle der Gruppe $-\overset{+}{N}$(Het)$Q^-$ eine Abgangsgruppe vorliegt,

mit einem Amin der Formel N(Het) umsetzt, oder

b) eine Verbindung der Formel

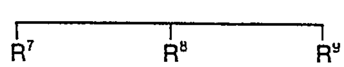

III

worin die Reste $R^7$, $R^8$ und $R^9$ die gleiche Bedeutung

tung wie die Reste $R^1$, $R^2$ bzw. $R^3$ haben, wobei jedoch an Stelle einer der Gruppen U und V eine Hydroxy- oder eine Aminogruppe vorliegt, mit einer Säure der Formel

$$Z^1-(A^1)_n-COOH \quad \text{oder} \quad Z^2-(A^2)_p-COOH \qquad IV$$

oder einem reaktiven Derivat davon, oder mit einem Isocyanat der Formel

$$Z^1NCO \quad \text{oder} \quad Z^2NCO \qquad V$$

oder einem Imidazolid der Formel

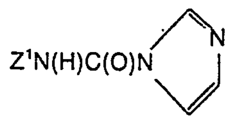
oder
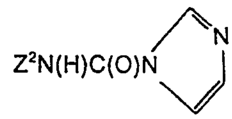
VI

worin $Y^1$, $Y^2$, $Z^1$, $Z^2$, $A^1$, $A^2$, T, N(Het), $Q^-$, n und p die oben angegebene Bedeutung haben, umsetzt.

Beispiele von in den Verbindungen der Formel II enthaltenen Abgangsgruppen sind Chlor, Brom, Jod, Mesyloxy, Phenylsulfonyloxy und Tosyloxy. Die Reaktion einer Verbindung II mit einem Amin N(Het) kann bei einer Temperatur bis zu Rückflusstemperatur des Reaktionsgemisches, zweckmässig bei etwa 80 °C, gegebenenfalls in einem Lösungsmittel, wie Acetonitril, Nitromethan oder einem aromatischen Kohlenwasserstoff, z. B. Toluol oder Benzol, durchgeführt werden.

Beispiele von reaktiven Derivaten der Säuren der Formel IV sind Säurebromide oder Säurechloride und Anhydride. Die Reaktion einer solchen Säure oder eines ihrer reaktiven Derivate mit einer Verbindung der Formel III kann in an sich bekannter Weise durchgeführt werden. Ein Säurechlorid oder Säurebromid kann in einem Lösungsmittel in Gegenwart einer Base bei einer Temperatur von etwa 0 bis 80 °C mit der Verbindung III umgesetzt werden. Ein Anhydrid kann in Gegenwart eines Katalysators, wie Dimethylaminopyridin, zweckmässig in einem Lösungsmittel mit der Verbindung III umgesetzt werden. Als Lösungsmittel können halogenierte Kohlenwasserstoffe, wie Chloroform oder Dichloräthan, und als Basen organische Basen, wie Triäthylamin, Chinolin oder Pyridin, oder anorganische Basen, wie Alkali- oder Erdalkalimetallhydroxyde, -carbonate oder -bicarbonate, z. B. $Na_2CO_3$, $KHCO_3$ oder $Ca_2CO_3$, verwendet werden.

Die Reaktion einer Verbindung der Formel III mit einem Isocyanat der Formel V oder mit einem entsprechenden Imidazolid der Formel VI kann in einem Lösungsmittel, wie Chloroform, Aceton oder Dimethylformamid, bei einer Temperatur zwischen etwa 0 und 100 °C, vorzugsweise bei etwa 40–60 °C, zweckmässig in Gegenwart eines Katalysators, wie einer Lewis-Base, z. B. Triäthylamin oder Diisopropyläthylamin, durchgeführt werden. Die Reaktion kann gegebenenfalls auch ohne Zusatz eines Lösungsmittels durchgeführt werden.

Die Verbindungen der Formel II können aus denjenigen der Formel VIII oder IX, und die Verbindungen der Formel III aus denjenigen der Formel VII nach dem folgenden Reaktionsschema hergestellt werden:

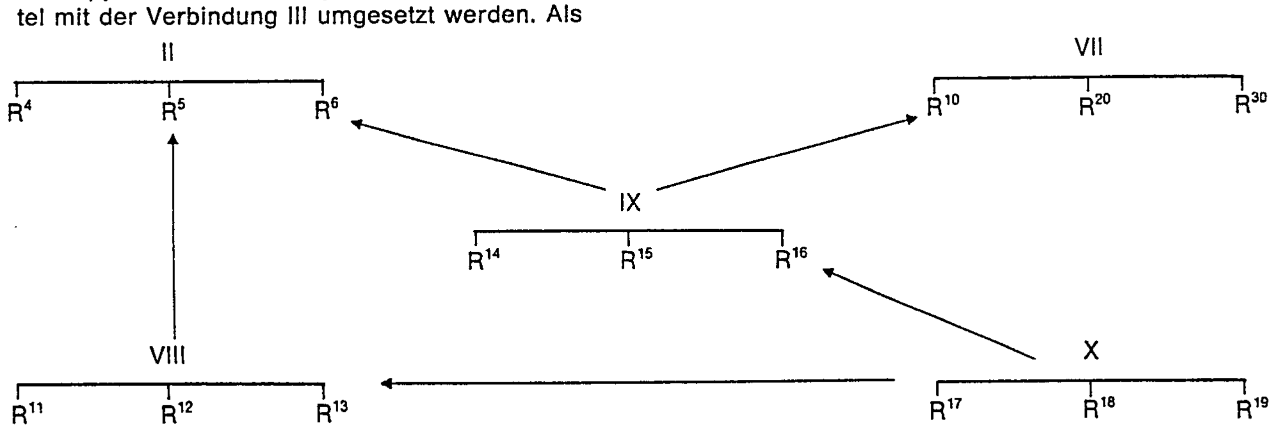

In den Verbindungen der Formel VII haben die Reste $R^{10}$, $R^{20}$ und $R^{30}$ die gleiche Bedeutung wie die Reste $R^1$, $R^2$ und $R^3$, wobei jedoch an Stelle einer der Gruppen U oder V eine gegebenenfalls geschützte Hydroxy- oder Aminogruppe oder eine Azidogruppe vorliegt.

In den Verbindungen der Formel VIII haben die Reste $R^{11}$, $R^{12}$ und $R^{13}$ die gleiche Bedeutung wie die Reste $R^1$, $R^2$ und $R^3$, wobei jedoch an Stelle der Gruppe W eine gegebenenfalls geschützte Hydroxy- oder Aminogruppe oder eine Azidogruppe vorliegt.

In den Verbindungen der Formel IX ist einer der Reste $R^{14}$, $R^{15}$ und $R^{16}$ eine gegebenenfalls geschützte Hydroxy- oder Aminogruppe oder eine Azidogruppe, ein weiterer ist eine Gruppe U oder V, und der verbleibende ist eine Gruppe W worin an Stelle von $\overset{+}{N}(Het)Q^-$ eine Abgangsgruppe vorliegt.

In den Verbindungen der Formel X ist einer der Reste $R^{17}$, $R^{18}$ und $R^{19}$ eine gegebenenfalls geschützte Hydroxy- oder Aminogruppe oder eine Azidogruppe, ein weiterer ist eine gegebenenfalls geschützte Hydroxygruppe, und der verbleibende ist eine Gruppe U oder V.

Beispiele von geschützten Hydroxy- und Aminogruppen sind Äthergruppen, wie Benzyloxy, Trityloxy oder 2-Tetrahydropyranyloxy, bzw. Succinimid, Phthalimid, Benzyloxycarbonylamino oder t-Butoxycarbonylamino.

Zur Herstellung einer Verbindung der Formel II kann man eine Verbindung der Formel VIII, worin z.B. $R^{13}$ Hydroxy ist, mit einem Halogenid der Formel Hal-T-($C_{2-6}$-Alkylen)-R, worin R eine Abgangsgruppe, Hal ein Halogenatom ist und T die obige Bedeutung hat, in Gegenwart einer Base, wie Pyridin, oder mit einem Isocyanat der Formel O=C=N-($C_{2-6}$-Alkylen)-R umsetzen.

Alternativ kann man eine Verbindung der Formel IX, worin z.B. $R^{14}$ Hydroxy ist, in die entsprechende Verbindung der Formel II, worin $R^4$ eine Gruppe U oder V ist, überführen, was in der gleichen Weise wie die weiter oben beschriebene Umwandlung einer Verbindung III in eine Verbindung I bewerkstelligt werden kann. Analog kann man eine Verbindung der Formel X, worin z.B. $R^{17}$ Hydroxy und $R^{19}$ eine geschützte Hydroxy- oder Aminogruppe ist, in die entsprechende Verbindung der Formel VIII, worin $R^{11}$ eine Gruppe U oder V ist, überführen.

Eine in einer Verbindung der Formel VIII enthaltene Azidogruppe oder geschützte Hydroxy- oder Aminogruppe, z.B. $R^{13}$, kann in an sich bekannter Weise in die freie Hydroxy- oder Aminogruppe umgewandelt werden. Eine Benzylgruppe kann durch Hydrogenolyse, z.B. über Palladium, abgespalten werden, eine Tritylgruppe mittels Trifluoressigsäure oder verdünnter Salzsäure, und eine 2-Tetrahydropyranylgruppe mittels verdünnter Salzsäure. Eine Azidogruppe kann mit einem komplexen Hydrid, wie Lithiumaluminiumhydrid, oder mittels $H_2$/Pd-C in die Aminogruppe übergeführt werden. Analog kann eine in einer Verbindung der Formel VII, IX oder X enthaltene Azidogruppe oder geschützte Hydroxy- oder Aminogruppe in die freie Hydroxy- oder Aminogruppe umgewandelt werden. Auf diese Weise wird eine Verbindung der Formel VII, worin z.B. $R^{10}$ eine geschützte Hydroxy- oder Aminogruppe ist, in die entsprechende Verbindung der Formel III, worin $R^7$ Hydroxy ist, übergeführt.

Zur Herstellung einer Verbindung der Formel IX, worin z.B. $R^{16}$ eine Gruppe W ist, in der eine Abgangsgruppe an Stelle von $-\overset{+}{N}(Het)Q^-$ vorliegt, kann man die entsprechende Verbindung der Formel X, worin $R^{19}$ Hydroxy ist, in der gleichen Weise behandeln wie weiter oben für die Überführung einer Verbindung VIII in eine solche der Formel II beschrieben.

Die Überführung einer Verbindung der Formel IX in eine solche der Formel VII kann in der gleichen Weise bewerkstelligt werden wie die Überführung einer Verbindung II in eine Verbindung I.

Die Verbindungen der Formeln II und III sind neu und als solche Bestandteil der Erfindung. Dies gilt auch für die Verbindungen der Formel VII, worin, falls $R^{10}$ eine gegebenenfalls geschützte Hydroxygruppe oder eine Gruppe $OY^1$ oder $OY^2$ und gleichzeitig $R^{20}$ eine gegebenenfalls geschützte Hydroxy- oder Aminogruppe, eine Azidogruppe oder eine Gruppe $OY^1$ oder V, und $X^3$ Sauerstoff ist, T eine Gruppe C(O)O oder C(O)NH sein soll.

Im Gegensatz zu den einen heterocyclischen Rest $-\overset{+}{N}$(Het) enthaltenden Verbindungen der Formeln I und VII enthalten die in der DE-A-2 820 892 und der EP-A-0 094 586 beschriebenen Verbindungen eine Amino-, Amido- oder Trialkylammoniumgruppe. Die vor dem Anmeldetag der vorliegenden Anmeldung hinterlegte jedoch nach diesem Anmeldetag veröffentlichte EP-A-0 146 258 beschreibt Verbindungen, die zwar einen heterocyclischen Rest enthalten, jedoch von den Verbindungen der Formeln I und VII strukturell abgegrenzt sind.

Die Verbindungen der Formel I und ihre Hydrate hemmen den Blutplättchenaktivierungsfaktor (PAF) und können bei der Bekämpfung bzw. Verhütung von Krankheiten, wie Thrombose, Apoplexie, Herzinfarkt, Angina pectoris, Bluthochdruck und durch Allergie verursachtes Bronchialasthma, und ferner als Entzündungshemmer und Antirheumatika verwendet werden.

Die Hemmwirkung auf PAF kann wie folgt nachgewiesen werden:

Plättchenreiches Plasma (PRP) wurde durch Zentrifugation von 1/10 Volumen 90 mM Trinatriumcitrat enthaltendem Kaninchen-Blut hergestellt. Die Aggregation der Blutplättchen wurde bei 37 °C unter Rühren mit Hilfe eines Aggregometers gemessen. 2 Minuten nach Zugabe der Testsubstanz zum PRP wurde die Plättchenaggregation durch eine submaximale Dosis von PAF (4 nM) ausgelöst. Der in der nachstehenden Tabelle angegebene $IC_{50}$-Wert (μM) entspricht derjenigen Konzentration der Testsubstanz, die die durch PAF ausgelöste Aggregation der Blutplättchen auf die Hälfte herabsetzt.

| Produkt von Beispiel: | 1 | 3 | 7a | 7b | 10 | 11 | 12 | 15 | 16 |
|---|---|---|---|---|---|---|---|---|---|
| $IC_{50}$ (μM) | 1,6 | 3 | 2 | 1,6 | 3 | 0,6 | 2 | 3 | 1,5 |

| Produkt von Beispiel: | 20b | 21a | 21b | 21d | 21e | 21f | 24 | 26 |
|---|---|---|---|---|---|---|---|---|
| $IC_{50}$ (μM) | 0,25 | 0,15 | 0,2 | 0,2 | 0,04 | 0,06 | 0,15 | 0,4 |

Wie eingangs erwähnt, sind Arzneimittel, enthaltend eine Verbindung der Formel I oder ein Hydrat davon, ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I oder ein Hydrat davon und erwünschtenfalls einen oder mehrere andere therapeutisch wertvolle Stoffe in eine galenische Darreichungsform bringt.

Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Öle, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Öle, und für Suppositorien eignen sich z.B. natürliche oder gehärtete Öle, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Drukkes, Puffer, Überzugsmittel oder Antioxidantien enthalten.

Die Dosierung des Wirkstoffes kann innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

Beispiel 1

A. Herstellung des Ausgangsmaterials

a) Eine Lösung von 0,5 g (1,15 mMol) (S)-2-O-Benzyl-1-O-octadecylglycerin in 25 ml Dichlormethan wird mit 0,3 ml Pyridin versetzt und auf 0 °C abgekühlt. Die Lösung wird mit 0,2 ml (1,99 mMol) Chlorameisensäure-2-bromäthylester versetzt, bei Raumtemperatur gerührt, mit 10 ml Wasser versetzt und mit 1N HCl auf pH 3 angesäuert. Die organische Phase wird mit Wasser gewaschen, getrocknet und eingedampft. Der Rückstand wird unter Eluieren mit Äther-n-Hexan (1 : 1) an Kieselgel chromatographiert. Man erhält das
(R)-2-O-Benzyl-1-[(2-bromäthoxy)carbonyl]-3-O-octadecylglycerin
in Form eines Öls.

b) Eine Lösung von
(R)-2-O-Benzyl-1-[(2-bromäthoxy)carbonyl]-3-O-octadecylglycerin
in Eisessig wird mit Palladiumoxyd und Wasserstoff versetzt. Der Katalysator wird abgenutscht uns das Filtrat wird unter vermindertem Druck getrocknet. Man erhält das (R)-1-[(2-Bromäthoxy)-carbonyl]-3-O-octadecylglycerin vom Schmelzpunkt 64–65 °C (Petroläther).

c) Eine Lösung von 0,26 g (0,52 mMol) (R)-1-[(2-Bromäthoxy)carbonyl]-3-O-octadecylglycerin in 5 ml Dichlormethan wird mit einer Lösung von 1 ml (10,5 mMol) Acetanhydrid und 0,050 g (0,45 mMol) N,N-Dimethylaminopyridin versetzt. Das Reaktionsgemisch wird mit gesättigter Natriumbicarbonatlösung versetzt und mit Dichlormethan extrahiert. Die organische Phase wird mit Wasser gewaschen, getrocknet und eingedampft. Durch Chromatographie an Kieselgel unter Eluieren mit n-Hexan-Äther (1 : 1) erhält man das
(R)-2-O-Acetyl-1-O-(2-bromäthoxy)-3-O-octadecylglycerin
in Form eines Öls.

B. Herstellung des Produktes

Eine Lösung von 0,37 g (0,688 mMol)
(R)-2-O-Acetyl-1-O-(2-bromäthoxy)-3-O-octadecylglycerin
in 10 ml Pyridin wird auf 80 °C während 3 Stunden erhitzt. Die Lösung wird abgedampft und der Rückstand wird durch azeotrope Destillation mit Toluol behandelt. Der Rückstand wird aus Aceton-Äther umkristallisiert. Man erhält das
1-[2-[[[(R)-2-O-Acetyl-3-(octadecyloxy)propoxy]carbonyl]oxy]äthyl]pyridinium-bromid
vom Schmelzpunkt 55–60 °C (Zers.).

Beispiel 2

A. Herstellung des Ausgangsmaterials

a) Eine Lösung von
(R)-1-O-Octadecyl-3-O-tritylglycerin
in Dichloräthan wird bei 80 °C mit Methylisocyanat versetzt. Die Lösung wird eingedampft und der Rückstand wird unter Eluieren mit Äther an Kieselgel chromatographiert. Man erhält das (R)-2-O-(Methylcarbamoyl)-1-O-octadecyl-3-O-tritylglycerin in Form eines Öls.

b) Eine Lösung von 0,57 g (0,91 mMol)
(R)-2-O-(Methylcarbamoyl)-1-O-octadecyl-3-O-tritylglycerin
in 15 ml Dichlormethan wird mit 0,5 ml Trifluoressigsäure versetzt. Die Lösung wird mit Wasser und mit Natriumbicarbonat gewaschen, getrocknet und eingedampft. Der Rückstand wird aus Dichlormethan-n-Hexan umkristallisiert. Man erhält das (S)-2-O-(Methylcarbamoyl)-1-O-octadecylglycerin vom Schmelzpunkt 68–69 °C.

c) In zu Beispiel 1 A.a) analoger Weise wird das
(S)-2-O-(Methylcarbamoyl)-1-O-octadecylglycerin
in das (R)-1-O-[(2-Bromäthoxy)carbonyl]-2-O-(methylcarbamoyl)-3-O-octadecylglycerin
vom Schmelzpunkt 62–65 °C übergeführt.

B. Herstellung des Produktes

Eine Lösung von 0,15 g (0,27 mMol)
(R)-1-O-[(2-Bromäthoxy)carbonyl]-2-O-(methylcarbamoyl)-3-O-octadecylglycerin
in 5 ml Pyridin wird bei 60 °C während 20 Stunden erhitzt. Die Lösung wird abgedampft und der Rückstand wird durch azeotrope Destillation mit Toluol behandelt. Der Rückstand wird aus Aceton umkristallisiert. Man erhält das
1-[2-[[[(R)-2-[(Methylcarbamoyl)oxy]-3-(octa-

decyloxy)propoxy]carbonyl]oxy]äthyl]pyridinium-bromid
vom Schmelzpunkt 94 °C (Zers.).

Beispiel 3

In zu Beispiel 2 analoger Weise erhält man unter Verwendung von Thiazol anstatt Pyridin das
3-[2-[[[(R)-2-[(Methylcarbamoyl)oxy]-3-(octadecyloxy)propoxy]carbonyl]oxy]äthyl]thiazolium-bromid
vom Schmelzpunkt 75 °C (Zers.).

Beispiel 4

A. Herstellung des Ausgangsmaterials

Eine Lösung von 0,17 g (0,474 mMol) (S)-2-O-Methyl-1-O-octadecylglycerin in 10 ml Dichloräthan wird mit 1 ml (11,63 mMol) 2-Chloräthylisocyanat versetzt und während 24 Stunden bei 80 °C erhitzt. Die Lösung wird abgedampft und der Rückstand wird an Kieselgel chromatographiert. Nach Eluieren mit Dichlormethan-Äther (9 : 1) erhält man das
(R)-1-O-[(2-Chloräthyl)carbamoyl]-2-O-methyl-3-O-octadecylglycerin in Form eines Öls.

B. Herstellung des Produktes

Eine Lösung von 0,035 g (0,075 mMol)
(R)-1-O-[(2-Chloräthyl)carbamoyl]-2-O-methyl-3-O-octadecylglycerin
wird mit 5 ml Pyridin versetzt und während 3 Tagen bei 80 °C erhitzt. Die Lösung wird abgedampft und der Rückstand wird durch azeotrope Destillation mit Toluol behandelt. Der Rückstand wird aus Aceton umkristallisiert. Man erhält das
1-[2-[1-[(R)-2-Methoxy-3-(octadecyloxy)propoxy]formamido]äthyl]pyridinium-chlorid
vom Schmelzpunkt 65 °C (Zers.).

Beispiel 5

A. Herstellung des Ausgangsmaterials

In zu Beispiel 1 A.a) analoger Weise wird das
(S)-2-O-Methyl-1-O-octadecylglycerin in das
(R)-1-O-[(2-Bromäthoxy)carbonyl]-2-O-methyl-3-O-octadecylglycerin
in Form eines Öls übergeführt.

B. Herstellung des Produktes

In zu Beispiel 1 B. analoger Weise wird das
(R)-1-O-[(2-Bromäthoxy)carbonyl]-2-O-methyl-3-O-octadecylglycerin
in das 1-[2-[[[(R)-2-Methoxy-3-(octadecyloxy)-propoxy]carbonyl]oxy]äthyl]pyridinium-bromid
vom Schmelzpunkt 53 °C (Zers.) übergeführt.

Beispiel 6

In zu Beispiel 4 bzw. 5 analoger Weise wird ausgehend von dem
(RS)-2-O-Methyl-1-O-octadecylcarbamoylglycerin
a) das 1-[2-[1-[(RS)-2-Methoxy-3-(octadecylcarbamoyloxy)propoxy]formamido]äthyl]-pyridinium-chlorid,
Schmelzpunkt 59–60 °C (Äthylacetat) bzw.
b) das 1-[2-[[[(RS)-2-Methoxy-3-[(octadecylcarbamoyl)oxy]propoxy]carbonyl]oxy]äthyl]-pyridinium-bromid,
Schmelzpunkt 85–86 °C (Äthylacetat) erhalten.

Beispiel 7

A. Herstellung des Ausgangsmaterials

Eine Lösung von 0,3 g (0,745 mMol) (S)-2-O-(Methoxycarbonyl)-1-O-octadecylglycerin in 15 ml Dichloräthan wird mit 2 ml (23,36 mMol) 2-Chloräthylisocyanat versetzt und bei 80 °C während 24 Stunden erhitzt. Die Lösung wird abgedampft und der Rückstand wird aus Aceton-Äther umkristallisiert. Man erhält das (R)-1-O-[(2-Chloräthyl)carbamoyl]-2-O-(methoxycarbonyl)-3-O-octadecylglycerin vom Schmelzpunkt 77–78 °C.

B. Herstellung des Produktes

Eine Lösung von 0,07 g (0,137 mMol)
(R)-1-O-[(2-Chloräthyl)carbamoyl]-2-O-(methoxycarbonyl)-3-O-octadecylglycerin
in 10 ml Pyridin wird bei 80 °C während 3 Tagen erhitzt. Die Lösung wird abgedampft und der Rückstand wird aus Aceton-n-Hexan umkristallisiert. Man erhält das
1-[2-[1-[(R)-2-[(Methoxycarbonyl)oxy]-3-(octadecyloxy)propoxy]formamido]äthyl]pyridinium-chlorid
vom Schmelzpunkt 83 °C (Zers.).

Beispiel 8

In zu Beispiel 7 B. analoger Weise erhält man unter Verwendung von Thiazol anstatt Pyridin das
3-[2-[1-[(R)-2-[(Methoxycarbonyl)oxy]-3-(octadecyloxy)propoxy]formamido]äthyl]thiazolium-chlorid
vom Schmelzpunkt 72 °C.

Beispiel 9

A. Herstellung des Ausgangsmaterials

In zu Beispiel 1 A.a) analoger Weise erhält man aus dem
(S)-2-O-(Methoxycarbonyl)-1-O-octadecylglycerin
das (R)-1-O-[(2-Bromäthoxy)carbonyl]-2-O-(methoxycarbonyl)-3-O-octadecylglycerin
in Form eines Öls.

B. Herstellung des Produktes

In zu Beispiel 5 B. analoger Weise erhält man aus dem
(R)-1-O-[(2-Bromäthoxy)carbonyl]-2-O-(methoxycarbonyl)-3-O-octadecylglycerin das 1-[2-[[(R)-2-[(Methoxycarbonyl)oxy]-3-(octadecyloxy)-propoxy]carbonyl]oxy]äthyl]pyridinium-bromid
vom Schmelzpunkt 84–85 °C (Zers.).

Beispiel 10

In zu Beispiel 9 B. analoger Weise erhält man aus dem
(R)-1-O-[(2-Bromäthoxy)carbonyl]-2-O-(methoxycarbonyl)-3-O-(octadecylcarbamoyl)glycerin
das 1-[2-[[[(R)-2-[(Methoxycarbonyl)oxy]-3-(octadecylcarbamoyl)oxy]propoxy]carbonyl]oxy]-äthyl]pyridinium-bromid
vom Schmelzpunkt 54 °C (Zers.). Das Ausgangsmaterial kann in Analogie zu Beispiel 10 A. erhalten werden.

Beispiel 11

In zu Beispiel 9 B. analoger Weise erhält man unter Verwendung von Thiazol anstatt Pyridin das 3-[2-[[[(R)-2-[(Methoxycarbonyl)oxy]-3-(octadecyloxy)propoxy]carbonyl]oxy]äthyl]thiazolium-bromid vom Schmelzpunkt 147 °C (Zers.).

Beispiel 12

A. Herstellung des Ausgangsmaterials

a) In zu Beispiel 4 A. analoger Weise erhält man aus dem (S)-2-O-Benzyl-1-O-octadecylglycerin das (R)-2-O-Benzyl-1-O-[(2-chloräthyl)carbamoyl]-3-O-octadecylglycerin in Form eines Öls.

b) In zu Beispiel 1 A.b) analoger Weise erhält man aus dem (R)-2-O-Benzyl-1-O-[(2-chloräthyl)carbamoyl]-3-O-octadecylglycerin das (R)-1-[(2-Chloräthyl)carbamoyl]-3-O-octadecylglycerin vom Schmelzpunkt 70 °C.

c) In zu Beispiel 2 A.a) analoger Weise erhält man aus dem (R)-1-[(2-Chloräthyl)carbamoyl]-3-O-octadecylglycerin das (R)-1-O-[(2-Chloräthyl)carbamoyl]-2-O-(methylcarbamoyl)-3-O-octadecylglycerin vom Schmelzpunkt 91–92 °C.

B. Herstellung des Produktes

In zu Beispiel 7 B. analoger Weise erhält man aus dem (R)-1-O-[(2-Chloräthyl)carbamoyl]-2-O-(methylcarbamoyl)-3-O-octadecylglycerin das 1-[2-[1-[(R)-(2-Methylcarbamoyl)-3-(octadecyloxy)propoxy]formamido]äthyl]pyridiniumchlorid, MS : $M^+$ = 550.

Beispiel 13

A. Herstellung des Ausgangsmaterials

a) Einer Lösung von 7,75 g (10,5 mMol) (RS)-1-O-Octadecyl-2-O-tosyl-3-O-tritylglycerin in 75 ml trockenem DMF wurden 2 g Natriumazid (30,8 mMol) zugegeben. Die Suspension wurde während 3 Stunden unter Feuchtigkeitsausschluss bei 100 °C gerührt. Nach Filtrierung der Feststoffe wurde das Lösungsmittel abdestilliert und der Rückstand wurde an Kieselgel mit Toluol-Pyridin (99 : 1 in Vol.) chromatographiert. Nach Kristallisation aus n-Hexan erhielt man 4,05 g (RS)-1-O-Octadecyl-2-deoxy-2-azido-3-O-tritylglycerin (63,3% der Theorie), Schmelzpunkt 58–59 °C.

b) Eine Lösung von 3,6 g (5,88 mMol) (RS)-1-O-Octadecyl-2-deoxy-2-azido-3-O-tritylglycerin in 20 ml trockenem Äther wurde unter Feuchtigkeitsausschluss zu einer Suspension von 130 mg $LiAlH_4$ (3,4 mMol) in 50 ml trockenem Äther getropft. Nach Ende der Entwicklung von $N_2$ wurde 10 Min. bei Raumtemperatur gerührt. Dann wurden Eiswürfel zugegeben und das Reaktionsgemisch 30 Minuten gerührt. Dann wurde die Ätherphase abgetrennt. Die feste Phase wurde mit Äther gewaschen und die vereinigten Ätherphasen wurden getrocknet. Nach Abdestillieren des Lösungsmittels wurde der Rückstand an einer Kieselgelsäule mit Äther-Methanol (9 : 1 in Vol.) chromatographiert. Nach Kristallisation aus n-Hexan wurden 3,1 g (RS)-1-O-Octadecyl-2-deoxy-2-amino-3-O-tritylglycerin erhalten (90,1% der Theorie), Schmelzpunkt 56–57 °C.

c) Einer auf 95 °C erwärmten Lösung von 4,65 g (RS)-1-O-Octadecyl-2-deoxy-2-amino-3-O-tritylglycerin (7,94 mMol) in 100 ml Dioxan wurden 5 ml 25%-iges wässriges HCl gegeben und das Reaktionsgemisch wurde während 30 Minuten bei 95 °C gehalten. Beim Abkühlen wurden 2,75 g (Ausbeute: 87,4%) (RS)-1-O-Octadecyl-2-deoxy-2-aminoglycerin-hydrochlorid, Schmelzpunkt 110–111 °C erhalten.

d) Einer Lösung von 2,75 g (RS)-1-O-Octadecyl-2-deoxy-2-aminoglycerin-hydrochlorid (7,24 mMol) in 30 ml Methanol wurden 1,4 g KOH (25 mMol) in 5 ml $H_2O$ unter Rühren zugetropft. Dann wurde das Methanol abdestilliert. Zum Rückstand wurden 10 ml $H_2O$ und 100 ml Dichlormethan gegeben. Zum Gemisch wurden unter Rühren 0,89 g Methylchloroformiat (9,4 mMol) getropft. Nach 1½ Stunden Rühren bei Raumtemperatur wurden die Phasen im Schütteltrichter getrennt. Die organische Phase wurde mit $H_2O$ gewaschen, getrocknet und nach Abdestillieren des Lösungsmittels und des Überschusses an Reagens wurde der Rückstand aus n-Hexan kristallisiert. Man erhielt 2,90 g (RS)-1-O-Octadecyl-2-deoxy-2-(1-methoxyformamido)glycerin (100% Ausbeute), Schmelzpunkt 63–64 °C.

e) Zu einer Lösung von 0,4 g (RS)-1-O-Octadecyl-2-deoxy-2-(1-methoxyformamido)glycerin (1 mMol) in 5 ml Chloroform und 0,25 ml Pyridin wurden im Eisbad 0,2 ml 2-Chloräthylchloroformiat (1,4 mMol) in 2 ml Chloroform unter Feuchtigkeitsausschluss zugetropft. Das Reaktionsgemisch wurde 2 Stunden bei Raumtemperatur gerührt. Nach Aufarbeitung des Reaktionsgemisches wurde über Kieselgel mit Äther filtriert und das Produkt aus n-Hexan kristallisiert. Es wurden 0,475 g (RS)-1-O-[(2-Chloräthoxy)carbonyl]-2-deoxy-2-(1-methoxyformamido)-3-O-octadecylglycerin erhalten (93,5% der Theorie), Schmelzpunkt 58–59 °C.

B. Herstellung des Produktes, 1-[2-[[[(RS)-2-(1-Methoxyformamido)-3-octadecyloxy]propoxy]carbonyloxy]äthyl]pyridiniumchlorid

0,3 g (0,59 mMol) (RS)-1-O-[(2-Chloräthoxy)carbonyl]-2-deoxy-2-(1-methoxyformamido)-3-O-octadecylglycerin wurden mit 5 ml trockenem Pyridin bei 80 °C 24 Stunden umgesetzt. Das Produkt wurde an Kieselgel mit Chloroform-Methanol (7 : 3) und dann mit

Chloroform-Methanol-Wasser (60 : 35 : 5) chromatographiert. Das in Methanol gelöste Produkt wurde dann einer Perkolation durch 10 ml eines Anionenaustauschers in der $Cl^-$ Form unterworfen. Man erhielt 125 mg einer beigen Verbindung (36% der Theorie), Schmelzpunkt 152–154 °C.

Beispiel 15

A. Herstellung des Ausgangsmaterials

0,75 g (1,87 mMol)
(RS)-1-O-octadecyl-2-deoxy-2-(1-methoxyformamido)glycerin
wurden mit 1 ml 2-Chloräthylisocyanat unter Feuchtigkeitsausschluss 2 Stunden bei 100 °C umgesetzt. Nach Beendigung der Reaktion wurde der Überschuss an Reagens abdestilliert und der Rückstand wurde aus n-Hexan kristallisiert. Man erhielt 0,88 g (92,8% der Theorie)
(RS)-1-O-[(2-Chloräthyl)carbamoyl]-2-deoxy-2-(1-methoxyformamido)-3-O-octadecylglycerin,
Schmelzpunkt 73–74 °C.

B. Herstellung des Produktes

In zu Beispiel 13 B. analoger Weise wurde das
(RS)-1-O-[(2-Chloräthyl)carbamoyl]-2-deoxy-2-(1-methoxyformamido)-3-O-octadecylglycerin
in das 1-[2-[1-[(RS)-2-(1-Methoxyformamido)-3-(octadecyloxy)propoxy]formamido]äthyl]pyridinium-chlorid,
Schmelzpunkt 195–197 °C übergeführt.

Beispiel 16

A. Herstellung des Ausgangsmaterials

a) In zu Beispiel 13 A.a) analoger Weise wurde das (RS)-1-O-Octadecyl-3-O-tosylglycerin
in das (RS)-1-O-Octadecyl-3-deoxy-3-azidoglycerin, Schmelzpunkt 42 °C (n-Hexan) übergeführt.

b) Eine Lösung von 5 g (RS)-1-O-Octadecyl-3-deoxy-3-azidoglycerin in 75 ml THF wurde mit 2,5 g 10%-iger Palladium-Kohle unter Normaldruck und bei Raumtemperatur hydriert. Nach 3 Stunden wurde der Katalysator abfiltriert, das Lösungsmittel wurde abdestilliert und das Produkt (RS)-1-O-Octadecyl-3-deoxy-3-aminoglycerin wurde aus Chloroform-Hexan kristallisiert. Man erhielt 4,1 g weisse Kristalle (88,2% der Theorie), Schmelzpunkt 68 °C.

c) Einer Lösung von 1,72 g (5 mMol) (RS)-1-O-Octadecyl-3-deoxy-3-aminoglycerin in 20 ml Dichlormethan wurden 2 ml einer wässrigen Lösung von 0,5 g KOH zugegeben. Unter Rührung des Zweiphasensystems wurden 0,7 ml 2-Bromäthylchloroformiat (entspricht 1,035 g bzw. 5,52 mMol) zugetropft. Nach einer Stunde Rühren bei Raumtemperatur wurden die Phasen getrennt. Die organische Phase wurde mit $H_2O$ gewaschen und getrocknet, das Lösungsmittel und der Überschuss an Reagens wurden abdestilliert und der Rückstand wurde aus n-Hexan kristallisiert. Man erhielt 2,1 g (84,8% der Theorie)
(RS)-1-O-Octadecyl-3-deoxy-3-[1-(2-brom)äthoxyformamido]glycerin, Schmelzpunkt 72–74 °C.

d) 0,5 g (1,01 mMol)
(RS)-1-O-Octadecyl-3-deoxy-3-[1-(2-brom)äthoxyformamido]glycerin
wurden mit 2 ml Methylisocyanat in Anwesenheit von 0,05 ml Diisopropyläthylamin bei 40 °C (Rückfluss) während 2 Stunden umgesetzt. Anschliessend wurde der Überschuss an Reagens abdestilliert und der Rückstand wurde aus n-Hexan kristallisiert. Es wurden 0,49 g (87,9% der Theorie)
(RS)-1-O-Octadecyl-2-O-(methylcarbamoyl)-3-deoxy-3-[1-(2-brom)äthoxyformamido]glycerin,
Schmelzpunkt 91–92 °C erhalten.

B. Herstellung des Produktes

Einer Lösung von 0,3 g (0,60 mMol)
(RS)-1-O-Octadecyl-2-O-(methylcarbamoyl)-3-deoxy-3-[1-(2-brom)äthoxyformamido]glycerin
in 1 ml Nitromethan wurden 2 ml trockenes Pyridin zugegeben. Man liess 24 Stunden bei 80 °C reagieren. Die Aufarbeitung und Reinigung wurde in Analogie zu Beispiel 14B. durchgeführt, mit der Ausnahme, dass der Ionenaustauscher in der $Br^-$ Form vorlag. Man erhielt 0,1 g (26,1% der Theorie)
1-[2-[[(RS)-2-[(Methylcarbamoyl)oxy]-3-(octadecyloxy)propyl]carbamoyl]äthyl]pyridinium-bromid,
Schmelzpunkt 195 °C (Zers.).

Beispiel 17

Es wurden hergestellt

a) analog Beispiel 2 das
1-[2-[[[(S)-[(Methylcarbonyl)oxy]-3-(octadecyloxy)propoxy]carbonyl]oxy]äthyl]pyridinium-bromid,
Smp. 96 °C (Zers.);

b) analog Beispiel 5 das
1-[2-[[[3-Methoxy-2(R)-(octadecyloxy)propoxy]carbonyl]oxy]äthyl]pyridinium-bromid,
Smp. 47 °C aus Aceton (Zers.);

c) analog Beispiel 7 das
1-[2-[1-[(R)-2-[(Methoxycarbonyl)oxy]-1-[(octadecyloxy)methyl]äthoxy]formamido]-äthyl]pyridinium-chlorid,
Smp. 57 °C

Beispiel 18

Analog Beispiel 6b wurden hergestellt

a) das
1-[3-[[(RS)-2-Methoxy-3-O-[(octadecylcarbamoyl)-oxy]propoxy]carbonyl]propyl]pyridinium-chlorid
in Form eines farblosen Wachses, MS : $M^+ = 549$;

b) das
3-[3-[[(RS)-2-Methoxy-3-[(octadecylcarbamoyl)-oxy]propoxy]carbonyl]propyl]thiazoliumchlorid,
Smp. 60–62 °C aus Äthylacetat.

Beispiel 19

Analog Beispiel 10 wurden hergestellt

a) das
1-[3-[[(RS)-2-[(Methoxycarbonyl)oxy]-3-[(octadecylcarbamoyl)oxy]propoxy]carbonyl]propyl]pyridinium-chlorid,
Smp. 51 °C;

b) das
1-[3-[[(R)-2-[(Methoxycarbonyl)oxy]-3-[(octade-

cylcarbamoyl)oxy]propoxy]carbonyl]-propyl]pyridinium-chlorid,
Smp. 55 °C;

c) das
1-[3-[[(S)-2-[(Methoxycarbonyl)oxy]-3-[[(octadecylcarbamoyl)oxy]propoxy]carbonyl]propyl]pyridinium-chlorid,
MS : $M^+$ = 593;

d) das
1-[3-[[(S)-2-[(Methoxycarbonyl)oxy]-3-[(octadecylcarbamoyl)oxy]propoxy]carbonyl]propyl]pyridinium-jodid,
MS : $M^+$ = 593;

e) das
3-[3-[[(R)-2-[(Methoxycarbonyl)oxy]-3-[(octadecylcarbamoyl)oxy]propoxy]carbonyl]-propyl]thiazolium-jodid,
Smp. 64 °C (Zers.);

f) das
3-[3-[[(S)-2-[(Methoxycarbonyl)oxy]-3-[(octadecylcarbamoyl)oxy]propoxy]carbonyl]-propyl]thiazolium-jodid,
Smp. 71 °C.

Beispiel 20

Analog obiger Beispiele wurde hergestellt das
1-[3-[[(RS)-2-[(Methylcarbonyl)oxy]-3-(octadecanoyloxy)propoxy]carbonyl]-propyl]pyridinium-chlorid
in Form eines Wachses, MS : $M^+$ = 564.

Beispiel 21

A. Herstellung des Ausgangsmaterials

a) 15,35 g (RS)-1-O-Benzyl-3-O-tritylglycerin (36,15 mM) (Helv. Chim. Acta 65, 1982, 1059) wurden in 75 ml Chloroform gelöst. 10 ml Pyridin wurden zugefügt, gefolgt von 10,5 g Tosylchlorid. Nach 24 Stunden bei Raumtemperatur wurde das Chloroform abdestilliert. Der Rückstand wurde in 50 ml Pyridin aufgenommen, 10 ml $H_2O$ und dann 10 g $KHCO_3$ wurden zugegeben. Nach Abdestillieren der Lösungsmittel wurde der Rückstand in Toluol aufgenommen, der feste Teil wurde abgetrennt, dann wurde die organische Phase mit $H_2O$ ausgeschüttelt, getrocknet und eingedampft. Das Produkt kristallisiert aus der Schmelze beim Abkühlen, Ausbeute 95%, Smp. 98–100 °C.

b) Analog Beispiel 13 Aa) bis 13 Ad) wurde das erhaltene
(RS)-1-O-Benzyl-2-O-tosyl-3-O-tritylglycerin
nacheinander in
das (RS)-1-O-Benzyl-2-deoxy-2-azido-3-O-tritylglycerin,
das (RS)-1-O-Benzyl-2-deoxy-2-amino-3-O-tritylglycerin,
Smp. 67–69 °C,
das (RS)-1-O-Benzyl-2-deoxy-2-aminoglycerin-hydrochlorid,
Smp. 148–149 °C, und
das (RS)-1-O-Benzyl-2-deoxy-2-(1-methoxyformamido)glycerin
übergeführt.

c) 0,45 g
(RS)-1-O-Benzyl-2-deoxy-2-(1-methoxyformamido)glycerin
(1,88 mM) wurden mit 0,6 g Octadecylisocyanat versetzt und die Lösung wurde 1 Stunde auf 90 °C erwärmt. Es wurde an Kieselgel mit einem Gemisch aus Toluol und Essigester (4 : 1) chromatographiert. Nach Kristallisieren aus n-Hexan erhielt man 0,65 g
(RS)-1-O-Benzyl-2-deoxy-2-(1-methoxyformamido)-3-O-(octadecylcarbamoyl)glycerin,
Smp. 65–67 °C.

d) 4,9 g
(RS)-1-O-Benzyl-2-deoxy-2-(1-methoxyformamido)-3-O-(octadecylcarbamoyl)glycerin
gelöst in 75 ml THF wurden in Anwesenheit von 1 g 10%iger Pd-Kohle, bei leichtem $H_2$-Überdruck hydriert. Es wurden 4,05 g
(RS)-2-Deoxy-2-(1-methoxyformamido)-1-O-octadecylcarbamoylglycerin erhalten,
Smp. 86 °C (aus n-Hexan).

e) Zu einer Lösung von 2 g
(RS)-2-Deoxy-2-(1-methoxyformamido)-1-O-octadecylcarbamoylglycerin
(4,5 mM) in 20 ml Chloroform und 0,7 ml Triäthylamin wurden im Eisbad 0,75 ml 4-Chlorbuttersäurechlorid (6,68 mM) in 5 ml Chloroform unter Feuchtigkeitsausschluss getropft. Das Reaktionsgemisch wurde 2 Stunden bei Raumtemperatur gerührt. Nach Aufarbeitung wurde das Reaktionsprodukt an Kieselgel mit Dichlormethan/Äther (1 : 1) filtriert. Es wurden 2,3 g
(RS)-1-O-(4-Chlorbutyryl)-2-deoxy-2-(1-methoxyformamido)-3-O-(octadecylcarbamoyl)-glycerin
nach Kristallisation aus n-Hexan erhalten, Smp. 68–70 °C.

B. Herstellung des Produktes

Analog Beispiel 13 B wurden aus 0,4 g des unter A.e) erhaltenen Chlorids 0,2 g
1-[3-[[(RS)-2-(1-Methoxyformamido)-3-(octadecylcarbamoyloxy)propoxy]carbonyl]propyl]pyridiniumchlorid
erhalten (44% der Theorie), Smp. 200 °C (Zers.).

Beispiel 22

Analog Beispiel 21 B wurde das
1-[3-[[(RS)-2-(1-Methoxyformamido)-3-(octadecylcarbamoyloxy)propoxy]carbonyl]propyl]-thiazoliumchlorid
erhalten, Smp. 180 °C (Zers.).

Beispiel 23

A. Herstellung des Ausgangsmaterials

a) Analog Beispiel 13 Aa) und b) wurde das (RS)-1-O-Tosyl-3-O-benzylglycerin (Helv. Chim. Acta 65, 1982, 1059) über das (RS)-1-Deoxy-1-azido-3-O-benzylglycerin in
das (RS)-1-Deoxy-1-amino-3-O-benzylglycerin,
Smp. 76–77 °C übergeführt.

b) 0,33 g
(RS)-1-Deoxy-1-amino-3-O-benzylglycerin
wurden in 20 ml Dichlormethan gelöst und mit

0,8 g Stearoylchlorid versetzt. Das Gemisch wurde in Anwesenheit einer wässrigen Lösung von KOH gerührt. Das aus der organischen Phase erhaltene
(RS)-1-Deoxy-1-octadecanamido-3-O-benzylglycerin
wurde an Kieselgel mit Äther chromatographiert. Nach Kristallisation aus n-Hexan erhielt man 0,5 g Kristalle, Smp. 72–73 °C.

c) 0,45 g
(RS)-1-Deoxy-1-octadecanamido-3-O-benzylglycerin
(1 mM) wurden mit 0,2 g Essigsäureanhydrid und 20 mg 4-Dimethylaminopyridin als Katalysator acetyliert. Nach Aufarbeitung, Filtration an Kieselgel mit Hexan/Äther (1 : 1) und Kristallisation in n-Hexan wurden 0,49 g
(RS)-1-Deoxy-1-octadecanamido-2-O-acetyl-3-O-benzylglycerin
erhalten, Smp. 53–55 °C.

d) Analog Beispiel 21 A.d) und e) wurde dieser Benzyläther über
das (RS)-1-Deoxy-1-octadecanamido-2-O-acetylglycerin,
Smp. 66–68 °C (aus n-Hexan) in
das (RS)-2-O-Acetyl-1-O-(4-chlorbutyryl)-3-deoxy-3-octadecanamidoglycerin
übergeführt, Smp. 55–56 °C.

B. Herstellung des Produktes

Analog Beispiel 13 B wurden aus 0,3 g
(RS)-2-O-Acetyl-1-O-(4-chlorbutyryl)-3-deoxy-3-octadecanamidoglycerin
0,16 g 1-[3-[[(RS)-2-Acetoxy-3-octadecanamidopropoxy]carbonyl]propyl]pyridiniumchlorid
erhalten (46% der Theorie), Smp. 220 °C (Zers.).

Beispiel 24

A. Herstellung der Ausgangsverbindung

a) Einer Lösung von 0,9 g
(RS)-1-Deoxy-1-amino-3-O-benzylglycerin
(5 mM) in 20 ml Dichlormethan wurde eine wässrige KOH-Lösung zugefügt. Dem Gemisch wurde unter Rühren eine Lösung von 1,7 g Octadecylchloroformiat in 10 ml Dichlormethan zugetropft. Nach 1 Stunde Rühren bei Raumtemperatur wurde aufgearbeitet und die erhaltene Verbindung an Kieselgel mit n-Hexan/Äther (1 : 1) chromatographiert. Nach Kristallisation aus n-Hexan erhielt man 1,6 g Kristalle, Smp. 58–59 °C.

b) 2,2 g
(RS)-1-Deoxy-1-[1-(octadecyloxy)formamido]-3-O-benzylglycerin
wurden mit Methylchloroformiat zu 2,15 g
(RS)-1-Deoxy-1-[1-(octadecyloxy)formamido]-2-O-methoxycarbonyl-3-O-benzylglycerin
acyliert, Smp. 52–54 °C (aus n-Hexan).

c) Analog Beispiel 21 Ad) und e) wurden aus dem erhaltenen Benzyläther über
das (RS)-1-Deoxy-1-[1-(octadecyloxy)formamido]-2-O-methoxycarbonylglycerin,
Smp. 56–57 °C (aus n-Hexan),
das (RS)-1-Deoxy-1-[1-(octadecyloxy)formamido]-2-O-methoxycarbonyl-3-O-(4-chlorbutyryl)glycerin
erhalten.

B. Herstellung des Produktes

Analog Beispiel 13 B wurden 0,4 g
(RS)-1-Deoxy-1-[1-(octadecyloxy)formamido]-2-O-methoxycarbonyl-3-O-(4-chlorbutyryl)-glycerin
in 0,15 g
1-[3-[[(RS)-2-[(Methoxycarbonyl)oxy]-3-[1-(octadecyloxy)formamido]propoxy]-carbonyl]propyl]pyridiniumchlorid
übergeführt (31,8% der Theorie), Smp. 168 °C.

Beispiel A

Eine Verbindung der Formel I kann wie folgt als Wirstoff zur Herstellung von Tabletten verwendet werden:

| | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch aus Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

Beispiel B

Eine Verbindung der Formel I kann wie folgt als Wirkstoff zur Herstellung von Kapseln verwendet werden:

| | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

**Patentansprüche für die Vertragsstaaten: BE, CH, DE, FR, GB, IT, LI, LU, NL, SE**

1. Glycerinderivate der Formel

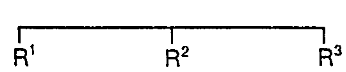

I

worin einer der Reste $R^1$, $R^2$ und $R^3$ eine Gruppe U der Formel $OY^1$ oder $X^1–C(O)–(A^1)_n–Z^1$

ein weiterer eine Gruppe V der Formel $OY^2$ oder $X^2–C(O)–A^2)_p–Z^2$

und der verbleibende eine Gruppe W der Formel $X^3T–(C_{2-6}\text{-Alkylen})–\overset{+}{N}(Het)Q^-$ ist,

wobei eines von $X^1$, $X^2$ und $X^3$ Sauerstoff oder NH ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

$A^1$ und $A^2$ Sauerstoff oder NH,

n und p die Zahl 1 oder 0,

$Z^1$ $C_{9-25}$-Alkyl oder $C_{9-25}$-Alkenyl,

$Z^2$ $C_{1-5}$-Alkyl, $C_{2-5}$-Alkenyl, Phenyl oder, falls $(A^2)_p$ nicht Sauerstoff ist, auch Wasserstoff bedeutet,

T Carbonyl, C(O)O oder C(O)NH oder, falls $X^3$ Sauerstoff ist, auch Methylen bedeutet, wobei, falls $R^1$ eine Gruppe $OY^1$ oder $OY^2$ und gleichzeitig $R^2$ eine Gruppe $OY^1$ oder V und $X^3$ Sauerstoff ist, T eine Gruppe C(O)O oder C(O)NH sein soll,

$–\overset{+}{N}(Het)$ ein gegebenenfalls durch Hydroxy, $C_{1-4}$-Alkyl, $C_{1-4}$-Alkoxy, 2-(Hydroxy oder Amino)-äthyl, Carbamoyl oder Ureido monosubstituierter Oxazolium-, Isoxazolium-, Pyridazinium-, Chinolinium-, Isochinolinium-, N-Methylimidazolium, Pyridinium- oder Thiazoliumrest, und $Q^-$ das Anion einer starken organischen oder anorganischen Säure ist,

und deren Hydrate.

2. Verbindungen nach Anspruch 1, worin $R^3$ eine Gruppe W ist.

3. Verbindungen nach Anspruch 1 oder 2, worin $R^1$ Octadecylcarbamoyloxy ist.

4. Verbindungen nach Anspruch 1, 2 oder 3, worin $R^2$ Methoxy oder Methoxycarbonyloxy ist.

5. Verbindungen nach Anspruch 1, 2, 3 oder 4, worin $R^3$

4-(1-Pyridiniumchlorid)-n-butyryloxy,

4-(1-Pyridiniumjodid)-n-butyryloxy,

4-(3-Thiazoliumchlorid)-n-butyryloxy

oder 4-(3-Thiazoliumjodid)-n-butyryloxy ist.

6. Verbindungen nach einem der Ansprüche 1–5, worin $R^1$ Octadecylcarbamoyloxy; $R^2$ Methoxy oder Methoxycarbonyloxy; und $R^3$ 4-(1-Pyridiniumchlorid)-n-butyryloxy, 4-(1-Pyridiniumjodid)-n-butyryloxy, 4-(3-Thiazoliumchlorid)-n-butyryloxy oder insbesondere 4-(3-Thiazoliumjodid)-n-butyryloxy ist.

7. Verbindungen nach einem der Ansprüche 1–6, worin $R^1$ Octadecylcarbamoyloxy, $R^2$ Methoxycarbonyloxy und $R^3$ 4-(3-Thiazoliumjodid)-n-butyryloxy ist.

8. 3-[3-[[(R)-2-(Methoxycarbonyloxy)-3-[(octadecylcarbamoyl)oxy]propoxy]carbonyl]propyl]thiazoliumjodid.

9. Eine Verbindung aus der Gruppe der folgenden

3-[3-[[(S)-2-[(Methoxycarbonyl)oxy]-3-[(octadecylcarbamoyl)oxy]propoxy]carbonyl]propyl]thiazoliumjodid,

1-[3-[[(RS)-2-[(Methoxycarbonyl)oxy]-3-[(octadecylcarbamoyl)oxy]propoxy]carbonyl]propyl]pyridiniumchlorid,

1-[3-[[(RS)-2-(1-Methoxyformamido-3-(octadecylcarbamoyloxy)propoxy]carbonyl]propyl]thiazoliumchlorid,

1-[3-[[(S)-2-[(Methoxycarbonyl)oxy]-3-[(octadecylcarbamoyl)oxy]propoxy]carbonyl]propyl]pyridiniumjodid,

1-[3-[[(R)-2-[(Methoxycarbonyl)oxy]-3-[(octadecylcarbamoyl)oxy]propoxy]carbonyl]propyl]pyridiniumchlorid und

3-[3-[[(RS)-2-Methoxy-3-[(octadecylcarbamoyl)oxy]propoxy]carbonyl]propyl]thiazoliumchlorid.

10. Glycerinderivate der Formel

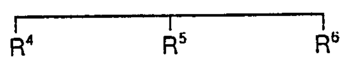

II

worin die Reste $R^4$, $R^5$ und $R^6$ die gleiche Bedeutung wie die Reste $R^1$, $R^2$ bzw. $R^3$ in Anspruch 1 haben, wobei jedoch an Stelle der Gruppe $N(Het)Q^-$ eine Abgangsgruppe vorliegt.

11. Glycerinderivate der Formel

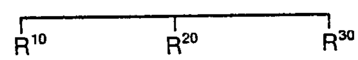

VII

worin die Reste $R^{10}$, $R^{20}$ und $R^{30}$ die gleiche Bedeutung wie die Reste $R^1$, $R^2$ bzw. $R^3$ in Anspruch 1 haben, wobei jedoch an Stelle einer der Gruppen U und V eine gegebenenfalls geschützte Hydroxy- oder Aminogruppe oder eine Azidogruppe vorliegt und worin, falls $R^{10}$ eine gegebenenfalls geschützte Hydroxygruppe oder eine Gruppe $OY^1$ oder $OY^2$ und gleichzeitig $R^{20}$ eine gegebenenfalls geschützte Hydroxy- oder Aminogruppe, eine Azidogruppe oder eine Gruppe $OY^1$ oder V, und $X^3$ Sauerstoff ist, T eine Gruppe C(O)O oder C(O)NH sein soll.

12. Verbindungen nach einem der Ansprüche 1–9 zur Verwendung als therapeutischer Wirkstoff.

13. Verbindung nach einem der Ansprüche 1–9 zur Verwendung als Hemmer des Blutplättchenaktivierungsfaktors (PAF).

14. Verfahren zur Herstellung der Verbindungen nach Anspruch 1, dadurch gekennzeichnet, dass man

a) eine Verbindung der Formel

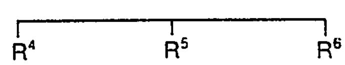

II

worin die Reste $R^4$, $R^5$ und $R^6$ die gleiche Bedeutung wie die Reste $R^1$, $R^2$ bzw. $R^3$ haben, wobei jedoch an Stelle der Gruppe $N(Het)Q^-$ eine Abgangsgruppe vorliegt,

mit einem Amin der Formel N(Het) umsetzt, oder

b) eine Verbindung der Formel

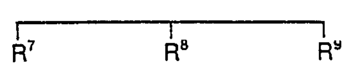

III

worin die Reste $R^7$, $R^8$ und $R^9$ die gleiche Bedeutung wie die Reste $R^1$, $R^2$ bzw. $R^3$ haben, wobei jedoch an Stelle einer der Gruppen U und V eine Hydroxy- oder eine Aminogruppe vorliegt,

mit einer Säure der Formel

$Z^1-(A^1)_n-COOH$ oder $Z^2-(A^2)_p-COOH$ IV

oder einem reaktiven Derivat davon, oder mit einem Isocyanat der Formel

$Z^1NCO$ oder $Z^2NCO$ V

oder einem Imidazolid der Formel

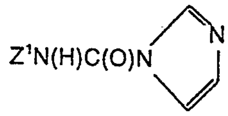 oder 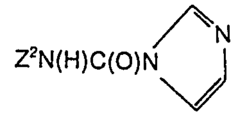 VI

worin $Y^1$, $Y^2$, $Z^1$, $Z^2$, $A^1$, $A^2$, T, N(Het), $Q^-$, n und p die oben angegebene Bedeutung haben, umsetzt.

15. Pharmazeutisches Präparat auf der Basis einer Verbindung nach Anspruch 1.

**Patentansprüche für den Vertragsstaat: AT**

1. Verfahren zur Herstellung von Glycerinderivaten der Formel

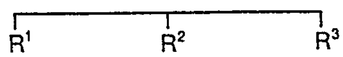 I

worin einer der Reste $R^1$, $R^2$ und $R^3$ eine Gruppe U der Formel $OY^1$ oder $X^1-C(O)-(A^1)_n-Z^1$

ein weiterer eine Gruppe V der Formel $OY^2$ oder $X^2-C(O)-(A^2)_p-Z^2$

und der verbleibende eine Gruppe W der Formel $X^3T-(C_{2-6}\text{-Alkylen})-\overset{+}{N}(Het)Q^-$ ist,

wobei eines von $X^1$, $X^2$ und $X^3$ Sauerstoff oder NH ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

$A^1$ und $A^2$ Sauerstoff oder NH,

n und p die Zahl 1 oder 0,

$Z^1$ $C_{9-25}$-Alkyl oder $C_{9-25}$-Alkenyl,

$Z^2$ $C_{1-5}$-Alkyl, $C_{2-5}$-Alkenyl, Phenyl oder, falls $(A^2)_p$ nicht Sauerstoff ist, auch Wasserstoff bedeutet,

T Carbonyl, C(O)O oder C(O)NH oder, falls $X^3$ Sauerstoff ist, auch Methylen bedeutet, wobei, falls $R^1$ eine Gruppe $OY^1$ oder $OY^2$ und gleichzeitig $R^2$ eine Gruppe $OY^1$ oder V und $X^3$ Sauerstoff ist, T eine Gruppe C(O)O oder C(O)NH sein soll,

$-\overset{+}{N}(Het)$ ein gegebenenfalls durch Hydroxy, $C_{1-4}$-Alkyl, $C_{1-4}$-Alkoxy, 2-(Hydroxy oder Amino)-äthyl, Carbamoyl oder Ureido monosubstituierter Oxazolium-, Isoxazolium-, Pyridazinium-, Chinolinium-, Isochinolinium-, N-Methylimidazolium, Pyridinium- oder Thiazoliumrest, und $Q^-$ das Anion einer starken organischen oder anorganischen Säure ist,

und von deren Hydraten, dadurch gekennzeichnet, dass man

a) eine Verbindung der Formel

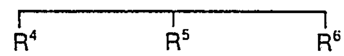 II

worin die Reste $R^4$, $R^5$ und $R^6$ die gleiche Bedeutung wie die Reste $R^1$, $R^2$ bzw. $R^3$ haben, wobei jedoch an Stelle der Gruppe $N(Het)Q^-$ eine Abgangsgruppe vorliegt,

mit einem Amin der Formel N(Het) umsetzt, oder

b) eine Verbindung der Formel

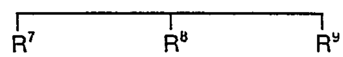 III

worin die Reste $R^7$, $R^8$ und $R^9$ die gleiche Bedeutung wie die Reste $R^1$, $R^2$ bzw. $R^3$ haben, wobei jedoch an Stelle einer der Gruppen U und V eine Hydroxy- oder eine Aminogruppe vorliegt,

mit einer Säure der Formel

$Z^1-(A^1)n-COOH$ oder $Z^2-(A^2)_p-COOH$ IV

oder einem reaktiven Derivat davon, oder mit einem Isocyanat der Formel

$Z^1NCO$ oder $Z^2NCO$ V

oder einem Imidazolid der Formel

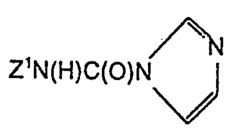 oder 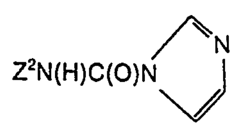 VI

worin $Y^1$, $Y^2$, $Z^1$, $Z^2$, $A^1$, $A^2$, T, N(Het), $Q^-$, n und p die oben angegebene Bedeutung haben, umsetzt.

2. Verfahren zur Herstellung von Verbindungen nach Anspruch 1, worin $R^3$ eine Gruppe W ist, dadurch gekennzeichnet, dass man entsprechend substituierte Ausgangsmaterialien einsetzt.

3. Verfahren zur Herstellung von Verbindungen nach Anspruch 1 oder 2, worin $R^1$ Octadecylcarbamoyloxy ist, dadurch gekennzeichnet, dass man entsprechend substituierte Ausgangsmaterialien einsetzt.

4. Verfahren zur Herstellung von Verbindungen nach Anspruch 1, 2 oder 3, worin $R^2$ Methoxy oder Methoxycarbonyloxy ist, dadurch gekennzeichnet, dass man entsprechend substituierte Ausgangsmaterialien einsetzt.

5. Verfahren zur Herstellung von Verbindungen nach Anspruch 1, 2, 3 oder 4, worin $R^3$
4-(1-Pyridiniumchlorid)-n-butyryloxy,
4-(1-Pyridiniumjodid)-n-butyryloxy,
4-(3-Thiazoliumchlorid)-n-butyryloxy
oder 4-(3-Thiazoliumjodid)-n-butyryloxy ist, dadurch gekennzeichnet, dass man entsprechend substituierte Ausgangsmaterialien einsetzt.

6. Verfahren zur Herstellung von Verbindungen nach einem der Ansprüche 1–5, worin $R^1$ Octadecylcarbamoyloxy; $R^2$ Methoxy oder Methoxycarbonyloxy; und $R^3$

4-(1-Pyridiniumchlorid)-n-butyryloxy,
4-(1-Pyridiniumjodid)-n-butyryloxy,
4-(3-Thiazoliumchlorid)-n-butyryloxy
oder 4-(3-Thiazoliumjodid)-n-butyryloxy ist, dadurch gekennzeichnet, dass man entsprechend substituierte Ausgangsmaterialien einsetzt.

7. Verfahren zur Herstellung von Verbindungen nach einem der Ansprüche 1–6, worin $R^1$ Octadecylcarbamoyloxy, $R^2$ Methoxycarbonyloxy und $R^3$ 4-(3-Thiazoliumjodid)-n-butyryloxy ist, dadurch gekennzeichnet, dass man entsprechend substituierte Ausgangsmaterialien einsetzt.

8. Verfahren zur Herstellung von
3-[3-[[(R)-2-(Methoxycarbonyloxy)-3-[(octadecylcarbamoyl)oxy]propoxy]carbonyl]propyl]thiazoliumjodid,
dadurch gekennzeichnet, dass man entsprechend substituierte Ausgangsmaterialien einsetzt.

9. Verfahren zur Herstellung einer Verbindung aus der Gruppe der folgenden
3-[3-[[(S)-2-[(Methoxycarbonyl)oxy]-3-[(octadecylcarbamoyl)oxy]propoxy]carbonyl]propyl]thiazoliumjodid,
1-[3-[[(RS)-2-[(Methoxycarbonyl)oxy]-3-[(octadecylcarbamoyl)oxy]propoxy]carbonyl]propyl]pyridiniumchlorid,
1-[3-[[(RS)-2-(1-Methoxyformamido-3-(octadecylcarbamoyloxy)propoxy]carbonyl]propyl]-thiazoliumchlorid,
1-[3-[[(S)-2-[(Methoxycarbonyl)oxy]-3-[(octadecylcarbamoyl)oxy]propoxy]carbonyl]propyl]pyridiniumjodid,
1-[3-[[(R)-2-[(Methoxycarbonyl)oxy]-3-[(octadecylcarbamoyl)oxy]propoxy]carbonyl]propyl]pyridiniumchlorid und
3-[3-[[(RS)-2-Methoxy-3-[(octadecylcarbamoyl)oxy]propoxy]carbonyl]propyl]thiazoliumchlorid,
dadurch gekennzeichnet, dass man entsprechend substituierte Ausgangsmaterialien einsetzt.

**Claims for the Contracting States: BE, CH, DE, FR, GB, IT, LI, LU, NL, SE**

1. Glycerine derivatives of the formula

$$R^1 \quad R^2 \quad R^3 \qquad \text{I}$$

wherein one of the residues $R^1$, $R^2$ and $R^3$ is a group U of the formula $OY^1$ or $X^1{-}C(O){-}(A^1)_n{-}Z^1$
another of the residues is a group V of the formula $OY^2$ or $X^2{-}C(O){-}(A^2)_p{-}Z^2$
and the remaining residue is a group W of the formula $X^3T{-}(C_{2-6}\text{-alkylene}){-}\overset{+}{N}(Het)Q^-$,
whereby one of $X^1$, $X^2$ and $X^3$ is oxygen or NH and the other two are oxygen,
$Y^1$ is $C_{10-26}$-alkyl or $C_{10-26}$-alkenyl,
$Y^2$ is $C_{1-6}$-alkyl, $C_{2-6}$-alkenyl, $C_{3-6}$-cycloalkyl, $C_{5-6}$-cycloalkenyl, phenyl, benzyl or 2-tetrahydropyranyl,
$A^1$ and $A^2$ are oxygen or NH,
n and p are the number 1 or 0,
$Z^1$ is $C_{9-25}$-alkyl or $C_{9-25}$-alkenyl,
$Z^2$ is $C_{1-5}$-alkyl, $C_{2-5}$-alkenyl, phenyl or, where $(A^2)_p$ is not oxygen, $Z^2$ also signifies hydrogen,
T is carbonyl, C(O)O or C(O)NH or, where $X^3$ is oxygen, T also signifies methylene, whereby, where $R^1$ is a group $OY^1$ or $OY^2$ and simultaneously $R^2$ is a group $OY^1$ or V and $X^3$ is oxygen, T must be a group C(O)O or C(O)NH,
$-\overset{+}{N}(Het)$ is an oxazolium, isoxazolium, pyridazinium, quinolinium, isoquinolinium, N-methylimidazolium, pyridinium or thiazolium residue optionally monosubstituted by hydroxy, $C_{1-4}$-alkyl, $C_{1-4}$-alkoxy, 2-(hydroxy or amino)-ethyl, carbamoyl or ureido, and $Q^-$ is the anion of a strong inorganic or organic acid,
and their hydrates.

2. Compounds according to claim 1, wherein $R^3$ is a group W.

3. Compounds according to claim 1 or 2, wherein $R^1$ is octadecylcarbamoyloxy.

4. Compounds according to claim 1, 2 or 3, wherein $R^2$ is methoxy or methoxycarbonyloxy.

5. Compounds according to claim 1, 2, 3 or 4, wherein $R^3$
is 4-(1-pyridinium chloride)-n-butyryloxy,
4-(1-pyridinium iodide)-n-butyryloxy,
4-(3-thiazolium chloride)-n-butyryloxy
or 4-(3-thiazolium iodide)-n-butyryloxy.

6. Compounds according to any one of claims 1–5, wherein $R^1$ is octadecylcarbamoyloxy; $R^2$ is methoxy or methoxycarbonyloxy; and $R^3$ is 4-(1-pyridinium chloride)-n-butyryloxy, 4-(1-pyridinium iodide)-n-butyryloxy, 4-(3-thiazolium chloride)-n-butyryloxy or especially 4-(3-thiazolium iodide)-n-butyryloxy.

7. Compounds according to any one of claims 1–6, wherein $R^1$ is octadecylcarbamoyloxy, $R^2$ is methoxycarbonyloxy and $R^3$ is 4-(3-thiazolium iodide)-n-butyryloxy.

8. 3-[3-[[(R)-2-(Methoxycarbonyloxy)-3-[(octadecylcarbamoyl)oxy]propoxy]carbonyl]propyl]thiazolium iodide.

9. A compound from the following group
3-[3-[[(S)-2-[(methoxycarbonyl)oxy]-3-[(octadecylcarbamoyl)oxy]propoxy]carbonyl]propyl]thiazolium iodide,
1-[3-[[(RS)-2-[(methoxycarbonyl)oxy]-3-[(octadecylcarbamoyl)oxy]propoxy]carbonyl]propyl]pyridinium chloride,
1-[3-[[(RS)-2-(1-methoxyformamido)-3-(octadecylcarbamoyloxy)propoxy]carbonyl]propyl]-thiazolium chloride,
1-[3-[[(S)-2-[(methoxycarbonyl)oxy]-3-[(octadecylcarbamoyl)oxy]propoxy]carbonyl]propyl]pyridinium iodide,
1-[3-[[(R)-2-[(methoxycarbonyl)oxy]-3-[(octadecylcarbamoyl)oxy]propoxy]carbonyl]propyl]pyridinium chloride and
3-[3-[[(RS)-2-methoxy-3-[(octadecylcarbamoyl)oxy]propoxy]carbonyl]propyl]-thiazolium chloride.

10. Glycerine derivatives of the formula

$$R^4 \quad R^5 \quad R^6 \qquad \text{II}$$

wherein the residues $R^4$, $R^5$ and $R^6$ have the same significance as the residues $R^1$, $R^2$ and $R^3$, respec-

tively, in claim 1, but in which a leaving group is present in place of the group $-N(Het)Q^-$.

11. Glycerine derivatives of the formula

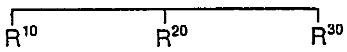 VII

wherein the residues $R^{10}$, $R^{20}$ and $R^{30}$ have the same significance as the residues $R^1$, $R^2$ and $R^3$, respectively, in claim 1, but in which an optionally protected hydroxy or amino group or an azido group is present in place of one of the groups U and V, and wherein, where $R^{10}$ is an optionally protected hydroxy group or a group $OY^1$ or $OY^2$ and simultaneously $R^{20}$ is an optionally protected hydroxy or amino group, an azido group or a group $OY^1$ or V and $X^3$ is oxygen, T must be a group C(O)O or C(O)NH.

12. Compounds according to any one of claims 1–9 for use as therapeutically active substances.

13. A compound according to claim 1 as inhibitors of blood platelet activating factor (PAF).

14. A process for the manufacture of the compounds according to claim 1, characterized by

a) reacting a compound of the formula

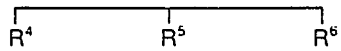 II

wherein the residues $R^4$, $R^5$ and $R^6$ have the same significance as the residues $R^1$, $R^2$ and $R^3$, respectively, but in which a leaving group is present in place of the group $-N(Het)Q^-$, with an amine of the formula N(Het), or

b) reacting a compound of the formula

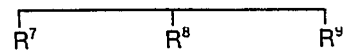 III

wherein the residues $R^7$, $R^8$ and $R^9$ have the same significance as the residues $R^1$, $R^2$ and $R^3$, respectively, but in which a hydroxy or an amino group is present in place of one of the groups U and V, with an acid of the formula

$$Z^1-(A^1)_n-COOH \quad \text{or} \quad Z^2-(A^2)_p-COOH \qquad IV$$

or a reactive derivatives thereof, or with an isocyanate of the formula

$$Z^1NCO \quad \text{or} \quad Z^2NCO \qquad V$$

or an imidazolide of the formula

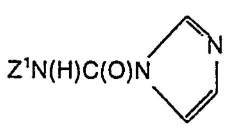 or 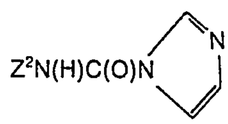 VI

wherein $Y^1$, $Y^2$, $Z^1$, $Z^2$, $A^1$, $A^2$, T, N(Het), $Q^-$ n and p have the significance given above.

15. A pharmaceutical preparation based on a compound according to claim 1.

**Claims for the Contracting State: AT**

1. A process for the manufacture of glycerine derivatives of the formula

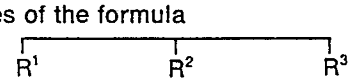 I

wherein one of the residues $R^1$, $R^2$ and $R^3$ is a group U of the formula $OY^1$ or $X^1-C(O)-(A^1)_n-Z^1$

another of the residues is a group V of the formula $OY^2$ or $X^2-C(O)-(A^2)_p-Z^2$

and the remaining residue is a group W of the formula $X^3T-(C_{2-6}\text{-alkylene})-\overset{+}{N}(Het)Q^-$, whereby one of $X^1$, $X^2$ and $X^3$ is oxygen or NH and the other two are oxygen,

$Y^1$ is $C_{10-26}$-alkyl or $C_{10-26}$-alkenyl,

$Y^2$ is $C_{1-6}$-alkyl, $C_{2-6}$-alkenyl, $C_{3-6}$-cycloalkyl, $C_{5-6}$-cycloalkenyl, phenyl, benzyl or 2-tetrahydropyranyl,

$A^1$ and $A^2$ are oxygen or NH,

n and p are the number 1 or 0,

$Z^1$ is $C_{9-25}$-alkyl or $C_{9-25}$-alkenyl,

$Z^2$ is $C_{1-5}$-alkyl, $C_{2-5}$-alkenyl, phenyl or, where $(A^2)_p$ is not oxygen, $Z^2$ also signifies hydrogen,

T is carbonyl, C(O)O or C(O)NH or, where $X^3$ is oxygen, T also signifies methylene, whereby, where $R^1$ is a group $OY^1$ or $OY^2$ and simultaneously $R^2$ is a group $OY^1$ or V and $X^3$ is oxygen, T must be a group C(O)O or C(O)NH,

$-\overset{+}{N}(Het)$ is an oxazolium, isoxazolium, pyridazinium, quinolinium, isoquinolinium, N-methylimidazolium, pyridinium or thiazolium residue optionally monosubstituted by hydroxy, $C_{1-4}$-alkyl, $C_{1-4}$-alkoxy, 2-(hydroxy or amino)-ethyl, carbamoyl or ureido, and $Q^-$ is the anion of a strong inorganic or organic acid,

and of their hydrates, characterized by

a) reacting a compound of the formula

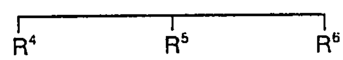 II

wherein the residues $R^4$, $R^5$ and $R^6$ have the same significance as the residues $R^1$, $R^2$ and $R^3$, respectively, but in which a leaving group is present in place of the group $-N(Het)Q^-$, with an amine of the formula N(Het), or

b) reacting a compound of the formula

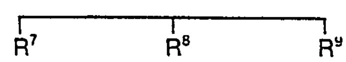 III

wherein the residues $R^7$, $R^8$ and $R^9$ have the same significance as the residues $R^1$, $R^2$ and $R^3$, respectively, but in which a hydroxy or an amino group is present in place of one of the groups U and V, with an acid of the formula

$$Z^1-(A^1)_n-COOH \quad \text{or} \quad Z^2-(A^2)_p-COOH \qquad IV$$

or a reactive derivatives thereof, or with an isocyanate of the formula

$$Z^1NCO \quad \text{or} \quad Z^2NCO \qquad V$$

or an imidazolide of the formula

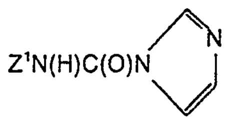 or 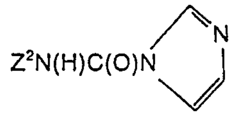 VI

wherein $Y^1$, $Y^2$, $Z^1$, $Z^2$, $A^1$, $A^2$, T, N(Het), $Q^-$ n and p have the significance given above.

2. A process for the manufacture of compounds according to claim 1 in which $R^3$ is a group W, characterized in that correspondingly substituted starting materials are used.

3. A process for the manufacture of compounds according to claim 1 or 2 in which $R^1$ is octadecylcarbamoyloxy, characterized in that correspondingly substituted starting materials are used.

4. A process for the manufacture of compounds according to claim 1, 2 or 3 in which $R^2$ is methoxy or methoxycarbonyloxy, characterized in that correspondingly substituted starting materials are used.

5. A process for the manufacture of compounds according to claim 1, 2, 3 or 4 in which $R^3$
is 4-(1-pyridinium chloride)-n-butyryloxy,
4-(1-pyridinium iodide)-n-butyryloxy,
4-(3-thiazolium chloride)-n-butyryloxy
or 4-(3-thiazolium iodide)-n-butyryloxy, characterized in that correspondingly substituted starting materials are used.

6. A process for the manufacture of compounds according to any one of claims 1–5 in which $R^1$ is octadecylcarbamoyloxy; $R^2$
is methoxy or methoxycarbonyloxy; and $R^3$
is 4-(1-pyridinium chloride)-n-butyryloxy,
4-(1-pyridinium iodide)-n-butyryloxy,
4-(3-thiazolium chloride)-n-butyryloxy,
or 4-(3-thiazolium iodide)-n-butyryloxy, characterized in that correspondingly substituted starting materials are used.

7. A process for the manufacture of compounds according to any one of claims 1–6 in which $R^1$ is octadecylcarbamoyloxy, $R^2$ is methoxycarbonyloxy and $R^3$ is 4-(3-thiazolium iodide)-n-butyryloxy, characterized in that correspondingly substituted starting materials are used.

8. A process for the manufacture of
3-[3-[[(R)-2-(methoxycarbonyloxy)-3-[(octadecylcarbamoyl)oxy]propoxy]carbonyl]propyl]thiazoliumiodide,
characterized in that correspondingly substituted starting materials are used.

9. A process for the manufacture of a compound from the following group
3-[3-[[(S)-2-[(methoxycarbonyl)oxy]-3-[(octadecylcarbamoyl)oxy]propoxy]carbonyl]propyl]thiazolium iodide,
1-[3-[[(RS)-2-[(methoxycarbonyl)oxy]-3-[(octadecylcarbamoyl)oxy]propoxy]carbonyl]propyl]pyridinium chloride,
1-[3-[[(RS)-2-(1-methoxyformamido)-3-(octadecylcarbamoyl)oxy]propoxy]carbonyl]propyl]-thiazolium chloride,
1-[3-[[(S)-2-[(methoxycarbonyl)oxy]-3-[(octadecylcarbamoyl)oxy]propoxy]carbonyl]propyl]pyridinium iodide,
1-[3-[[(R)-2-[(methoxycarbonyl)oxy]-3-[(octadecylcarbamoyl)oxy]propoxy]carbonyl]propyl]pyridinium chloride and
3-[3-[[(RS)-2-methoxy-3-[(octadecylcarbamoyl)oxy]propoxy]carbonyl]propyl]-thiazolium chloride,
characterized in that correspondingly substituted starting materials are used.

**Revendications pour les états contractants: BE, CH, DE, FR, GB, IT, LI, LU, NL, SE**

1. Dérivé de la glycérine de formule

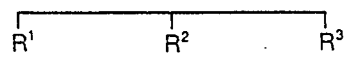 I

où un des groupes $R^1$, $R^2$ et $R^3$ est un groupe U de formule $OY^1$ ou $X^1$–C(O)–$(A^1)_n$–$Z^1$

un deuxième est un groupe V de formule $OY^2$ ou $X^2$–C(O)–$(A^2)_p$–$Z^2$

et le restant est un groupe W de formule $X^3$T-(alcoylène en $C_{2-6}$)–$\overset{+}{N}$(het)$Q^-$,

dans lequel un des $X^1$, $X^2$ et $X^3$ est l'oxygène ou NH et les deux autres sont de l'oxygène,

$Y^1$ représente un alkyle en $C_{10-26}$ ou un alcényle en $C_{10-26}$,

$Y^2$ représente un alkyle en $C_{1-6}$, un alcényle en $C_{2-6}$, un cycloalkyle en $C_{3-6}$, un cycloalcényle en $C_{5-6}$, le phényle, le benzyle ou le 2-tétrahydropyrannyle,

$A^1$ et $A^2$ représentent l'oxygène ou NH,

n et p représentent le chiffre 1 ou 0,

$Z^1$ représente un alkyle en $C_{9-25}$ ou un alcényle en $C_{9-25}$,

$Z^2$ représente un alkyle en $C_{1-5}$, un alcényle en $C_{2-5}$, le phényle ou aussi l'hydrogène, si $(A^2)_p$ n'est pas l'oxygène,

T représente le carbonyle, C(O)O ou C(O)NH ou aussi le méthylène, si $X^3$ est l'oxygène, dans lequel T doit être un groupe C(O)O ou C(O)NH, si $R^1$ est un groupe $OY^1$ ou $OY^2$ et si en même temps $R^2$ est un groupe $OY^1$ ou V et $X^3$ l'oxygène,

–$\overset{+}{N}$(het) représente un reste oxazolium, isoxazolium, pyridazinium, quinoléinium, isoquinoléinium, N-méthylimidazolium, pyridinium ou thiazolium, éventuellement monosubstitué par un hydroxy, un alkyle en $C_{1-4}$, un alcoxy en $C_{1-4}$, un 2-(hydroxy ou amino)-éthyle, un carbamoyle ou un uréido, et $Q^-$ est l'anion d'un acide fort organique ou minéral,

ou ses hydrates.

2. Composé selon la revendication 1, dans lequel $R^3$ est un groupe W.

3. Composé selon l'une quelconque des revendications 1 ou 2, dans lequel $R^1$ est l'octadécylcarbamoyloxy.

4. Composé selon l'une quelconque des revendications 1, 2 ou 3, dans lequel $R^2$ est le méthoxy ou le méthoxycarbonyloxy.

5. Composé selon l'une quelconque des revendications 1 à 4, dans lequel $R^3$ est le
4-(1-chlorure de pyridinium)-n-butyryloxy,
le 4-(1-iodure de pyridinium)-n-butyryloxy,
le 4-(3-chlorure de thiazolium)-n-butyryloxy,
ou le 4-(3-iodure de thiazolium)-n-butyryloxy.

6. Composé selon l'une quelconque des revendications 1 à 5, dans lequel $R^1$ est l'octadécylcarbamoyloxy; $R^2$ est le méthoxy ou le méthoxycarbonyloxy; et $R^3$ est le 4-(1-chlorure de pyridinium)-n-butyryloxy, le 4-(1-iodure de pyridinium)-n-butyryloxy, le 4-(3-chlorure de thiazolium)-n-butyryloxy ou en particulier le 4-(3-iodure de thiazolium)-n-butyryloxy.

7. Composé selon l'une quelconque des revendications 1 à 6, dans lequel $R^1$ est l'octadécylcarbamoyloxy, $R^2$ est le méthoxycarbonyloxy et $R^3$ est le 4-(3-iodure de thiazolium)-n-butyryloxy.

8. Iodure de
3-[3-[[(R)-2-(méthoxycarbonyloxy)-3-[(octadécylcarbamoyl)oxy]propoxy]carbonyl]propyl]thiazolium.

9. Composé choisi dans le groupe constitué par
l'iodure de
3-[3-[[(S)-2-[(méthoxycarbonyl)oxy]-3-[(octadécylcarbamoyl)oxy]propoxy]carbonyl]propyl]-thiazolium,
le chlorure de
1-[3-[[(RS)-2-[(méthoxycarbonyl)oxy]-3-[(octadécylcarbamoyl)oxy]propoxy]carbonyl]propyl]pyridinium,
le chlorure de
1-[3-[[(RS)-2-(1-méthoxyformamido-3-(octadécylcarbamoyloxy)propoxy]carbonyl]propyl]-thiazolium,
l'iodure de
1-[3-[[(S)-2-[(méthoxycarbonyl)oxy]-3-[octadécylcarbamoyl)oxy]propoxy]carbonyl]propyl]pyridinium,
le chlorure de
1-[3-[[(R)-2-[(méthoxycarbonyl)oxy]-3-[(octadécylcarbamoyl)oxy]propoxy]carbonyl]propyl]pyridinium et
le chlorure de
3-[3-[[(RS)-2-méthoxy-3-[(octadécylcarbamoyl)oxy]propoxy]carbonyl]propyl]-thiazolium.

10. Dérivé de la glycérine de formule

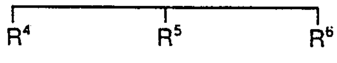 II

dans laquelle les restes $R^4$, $R^5$ et $R^6$ ont la même signification que les restes $R^1$, $R^2$ et $R^3$, respectivement, dans la revendication 1, dans lequel cependant se trouve un groupe de départ à la place du groupe $N(het)Q^-$.

11. Dérivé de la glycérine de formule

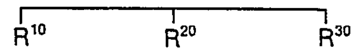 VII

dans laquelle les restes $R^{10}$, $R^{20}$ et $R^{30}$ ont la même signification que les restes $R^1$, $R^2$ et $R^3$, respectivement, dans la revendication 1, dans lequel cependant, au lieu d'un des groupes U et V se trouve un groupe hydroxy ou amino, éventuellement protégé, ou un groupe azido, et dans lequel T doit être un groupe C(O)O ou C(O)NH, si $R^{10}$ est un groupe hydroxy, éventuellement protégé, ou un groupe $OY^1$ ou $OY^2$ et en même temps $R^{20}$ est un groupe hydroxy ou amino, éventuellement protégé, un groupe azido ou un groupe $OY^1$ ou V, et si $X^3$ est l'oxygène.

12. Composé selon l'une quelconque des revendications 1 à 9 destiné à être utilisé comme produit thérapeutique.

13. Composé selon l'une quelconque des revendications 1 à 9 destiné à être utilisé comme inhibiteur du facteur d'activation des plaquettes du sang (PAF).

14. Procédé pour préparer le composé selon la revendication 1, caractérisé en ce que:

a) on transforme avec une amine de formule N(het) un composé de formule

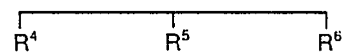 II

dans laquelle les restes $R^4$, $R^5$ et $R^6$ ont la même signification que les restes $R^1$, $R^2$ et $R^3$, respectivement, dans lequel cependant à la place du groupe $N(het)Q^-$ se trouve un groupe de départ, ou

b) on transforme un composé de formule

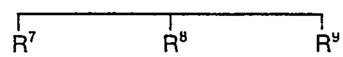 III

dans laquelle les restes $R^7$, $R^8$ et $R^9$ ont la même signification que les restes $R^1$, $R^2$ et $R^3$, respectivement, dans lequel cependant à la place d'un des groupes U et V se trouve un groupe hydroxy ou un groupe amino, avec un acide de formule

$$Z^1-(A^1)_n-COOH \quad \text{ou} \quad Z^2-(A^2)_p-COOH \qquad IV$$

ou un dérivé réactif de celui-ci, ou avec un isocyanate de formule

$$Z^1NCO \quad \text{ou} \quad Z^2NCO \qquad V$$

ou un imidazolide de formule

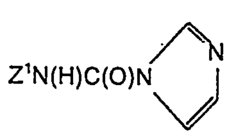 ou 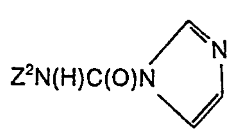 VI

dans laquelle $Y^1$, $Y^2$, $Z^1$, $Z^2$, $A^1$, $A^2$, T, N(het), $Q^-$, n et p ont la signification indiquée précédemment.

15. Préparation pharmaceutique à base d'un composé selon la revendication 1.

**Revendications pour l'état contractant: AT**

1. Procédé pour la fabrication de dérivés de la glycérine de formule

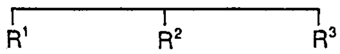

I

où un des restes $R^1$, $R^2$ et $R^3$ est un groupe U de formule $OY^1$ ou $X^1$–C(O)–$(A^1)_n$–$Z^1$

un deuxième est un groupe V de formule $OY^2$ ou $X^2$–C(O)–$(A^2)_p$–$Z^2$

et le restant est un groupe W de formule $X^3$T-(alcoylène en $C_{2-6}$)–$\overset{+}{N}$(Het)$Q^-$,

dans lequel un des $X^1$, $X^2$ et $X^3$ est l'oxygène ou NH et les deux autres sont de l'oxygène,

$Y^1$ représente un alkyle en $C_{10-26}$ ou un alcényle en $C_{10-26}$,

$Y^2$ représente un alkyle en $C_{1-6}$, un alcényle en $C_{2-6}$, un cycloalkyle en $C_{3-6}$, un cycloalcényle en $C_{5-6}$, le phényle, le benzyle ou le 2-tétrahydropyrannyle,

$A^1$ et $A^2$ représentent l'oxygène ou NH,

n et p représentent le chiffre 1 ou 0,

$Z^1$ représente un alkyle en $C_{9-25}$ ou un alcényle en $C_{9-25}$,

$Z^2$ représente un alkyle en $C_{1-5}$, un alcényle en $C_{2-5}$, le phényle ou aussi l'hydrogène, si $(A^2)_p$ n'est pas l'oxygène,

T représente le carbonyle, C(O)O ou C(O)NH ou aussi le méthylène, si $X^3$ est l'oxygène, dans lequel T doit être un groupe C(O)O ou C(O)NH, si $R^1$ est un groupe $OY^1$ ou $OY^2$ et si en même temps $R^2$ est un groupe $OY^1$ ou V et $X^3$ l'oxygène,

–$\overset{+}{N}$(het) représente un reste oxazolium, isoxazolium, pyridazinium, quinoléinium, isoquinoléinium, N-méthyl-imidazolium, pyridinium ou thiazolium, éventuellement monosubstitué avec un hydroxy, un alkyle en $C_{1-4}$, un alcoxy en $C_{1-4}$, un 2-(hydroxy ou amino)-éthyl, un carbamoyle ou un uréido, et $Q^-$ est l'anion d'un acide fort organique ou minéral,

et de leurs hydrates, caractérisé en ce que

a) on transforme avec une amine de formule N(het) un composé de formule

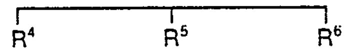

II

dans laquelle les restes $R^4$, $R^5$ et $R^6$ ont la même signification que les restes $R^1$, $R^2$ et $R^3$, respectivement, dans lequel cependant à la place du groupe N(het)$Q^-$ se trouve un groupe de départ, ou

b) on transforme un composé de formule

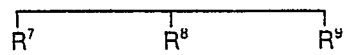

III

dans laquelle les restes $R^7$, $R^8$ et $R^9$ ont la même signification que les restes $R^1$, $R^2$ et $R^3$ respectivement, dans lequel cependant à la place d'un des groupes U et V se trouve un groupe hydroxy ou un groupe amino, avec un acide de formule

$Z^1$–$(A^1)_n$–COOH ou $Z^2$–$(A^2)_p$–COOH IV

ou un dérivé réactif de celui-ci, ou avec un isocyanate de formule

$Z^1$NCO ou $Z^2$NCO V

ou un imidazolide de formule

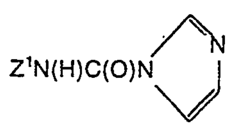

ou

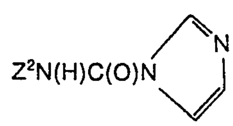

VI

dans lequel $Y^1$, $Y^2$, $Z^1$, $Z^2$, $A^1$, $A^2$, T, N(het), $Q^-$, n et p ont la signification donnée précédemment.

2. Procédé pour préparer le composé selon la revendication 1, dans lequel $R^3$ est un groupe W, caractérisé en ce que l'on emploie des matières de base substituées de façon correspondante.

3. Procédé pour préparer un composé selon l'une quelconque des revendications 1 ou 2, dans lequel $R^1$ est un octadécylcarbamoyloxy, caractérisé en ce que l'on utilise des matières de base substituées de façon correspondante.

4. Procédé pour préparer un composé selon l'une quelconque des revendications 1, 2 ou 3, dans lequel $R^2$ est un méthoxy ou un méthoxycarbonyloxy, caractérisé en ce que l'on utilise des matières de base substituées de façon correspondante.

5. Procédé pour préparer un composé selon l'une quelconque des revendications 1, 2, 3 ou 4, dans lequel $R^3$ est le
4-(1-chlorure de pyridinium)-n-butyryloxy,
le 4-(1-iodure de pyridinium)-n-butyryloxy,
le 4-(3-chlorure de thiazolium)-n-butyryloxy
ou le 4-(3-iodure de thiazolium)-n-butyryloxy, caractérisé en ce que l'on utilise des matières de base substituées de façon correspondante.

6. Procédé pour préparer un composé selon l'une quelconque des revendications 1 à 5, dans lequel $R^1$ est l'octadécylcarbamoyloxy; $R^2$ est le méthoxy ou un méthoxycarbonyloxy et $R^3$ est le
4-(1-chlorure de pyridinium)-n-butyryloxy,
le 4-(1-iodure de pyridinium)-n-butyryloxy,
le 4-(3-chlorure de thiazolium)-n-butyryloxy
ou le 4-(3-iodure de thiazolium)-n-butyryloxy, caractérisé en ce que l'on utilise des matières de base substituées de façon correspondante.

7. Procédé pour préparer un composé selon l'une quelconque des revendications 1 à 6, dans lequel $R^1$ est un octadécylcarbamoyloxy, $R^2$ est un méthoxycarbonyloxy et $R^3$ est un 4-(3-iodure de thiazolium)-n-butyryloxy, caractérisé en ce que l'on utilise des matières de base substituées de façon correspondante.

8. Procédé pour préparer l'iodure de
3-[3-[[(R)-2-(méthoxycarbonyloxy)-3-[(octadécylcarbamoyl)oxy]propoxy]carbonyl]propyl]thiazolium,

caractérisé en ce que l'on utilise des matières de base substituées de façon correspondante.

9. Procédé pour préparer un composé choisi dans le groupe constitué par les composés suivants: l'iodure de
3-[3-[[(S)-2-[(méthoxycarbonyl)oxy]-3-[(octadécylcarbamoyl)oxy]propoxy]carbonyl]propyl]thiazolium,
le chlorure de
1-[3-[[(RS)-2-[(méthoxycarbonyl)oxy]-3-[(octadécylcarbamoyl)oxy]propoxy]carbonyl]propyl]pyridinium,
le chlorure de
1-[3-[[(RS)-2-(1-méthoxyformamido-3-(octadécylcarbamoyloxy)propoxy]carbonyl]propyl]-thiazolium,
l'iodure de
1-[3-[[(S)-2-[(méthoxycarbonyl)oxy]-3-[(octadécylcarbamoyl)oxy]propoxy]carbonyl]propyl]pyridinium,
le chlorure de
1-[3-[[(R)-2-[(méthoxycarbonyl)oxy]-3-[(octadécylcarbamoyl)oxy]propoxy]carbonyl]propyl]pyridinium et
le chlorure de
3-[3-[[(RS)-2-méthoxy-3-[(octadécylcarbamoyl)-oxy]propoxy]carbonyl]propyl]thiazolium,
caractérisé en ce que l'on utilise des matières de base substituées de façon correspondante.